(19) Europäisches Patentamt
European Patent Office
Office européen des brevets

(11) **EP 0 761 159 B1**

## (12) EUROPEAN PATENT SPECIFICATION

(45) Date of publication and mention
of the grant of the patent:
**29.09.1999 Bulletin 1999/39**

(51) Int. Cl.$^6$: **A61B 5/00**, G01N 21/31

(21) Application number: **96112658.8**

(22) Date of filing: **06.08.1996**

(54) **Apparatus for medical monitoring, in particular pulse oximeter**

Medizinisches Überwachungsgerät, insbesondere Puls-Oximeter

Appareil de surveillance médicale, en particulier oximètre détecteur de pouls

(84) Designated Contracting States:
**DE FR GB**

(30) Priority: **31.08.1995 EP 95113654**

(43) Date of publication of application:
**12.03.1997 Bulletin 1997/11**

(73) Proprietor:
**Hewlett-Packard Company
Palo Alto, California 94304 (US)**

(72) Inventor: **Kästle, Siegfried
71154 Nufringen (DE)**

(74) Representative: **Kurz, Peter
Hewlett-Packard GmbH,
Europ. Patent- und Lizenzabteilung,
Herrenberger Strasse 130
71034 Böblingen (DE)**

(56) References cited:
EP-A- 0 102 816      EP-A- 0 502 717
WO-A-92/13598        US-A- 4 800 885
US-A- 5 349 953

**Description**

This invention relates to an apparatus for measuring medical parameters of a patient by irradiation of electromagnetic waves into a sample or material and for measurement and subsequent analysis of the electromagnetic waves which have passed through said sample or material, and to a related method. More specifically and in one particular embodiment, the invention relates to the field of pulse oximetry.

Optical methods for measuring medical parameters of a patient are well-known in the art. These include, for example, blood flow measurement, measurement of the perfusion, blood gas analysis, infrared gas analyzers etc., and in particular pulse oximetry. What is common to all of these methods is that light of the visible or adjoining spectra (such as infrared), or other electromagnetic waves, are irradiated into the tissue of a patient, into a sample taken from a patient (such as a blood sample) or into air inspired or expired by the patient. The light transmitted through, or reflected by such sample or material is then analyzed for changes of its characteristics, such as absorbance, wavelength etc., in order to determine a medical parameter of interest. It is understood that such methods are not limited to the use of electromagnetic waves of optical nature (i.e., in the visible spectrum), but that they may use other waves in the electromagnetic spectrum as well. Further, such methods may use the *in vivo*, as well as the *in vitro* approach, i.e., they may be focused on the analysis of the patient (e.g., its tissue), or on samples taken from a patient.

In order to increase the clarity and comprehensibility of this description, the underlying problems will now be explained by means of one specific example, namely pulse oximetry. However, it is understood that this does by no means limit the scope of the present invention which is, in fact, also applicable for the measurement of other medical parameters.

Pulse oximetry is a non-invasive technique to evaluate the condition of a patient. Usually, a sensor or a probe comprises light emitting means such as light-emitting diodes (LEDs). Two or more of these LEDs with different wavelengths (e.g., red and infrared) may be used. The emitted light is directed into the tissue of the patient, and light receiving means such as photodiodes or phototransistors measure the amount of transmitted or reflected light. In the case of transmission measurement, the transmitter and receiver diodes are arranged opposite to each other with respect to the human tissue, whereas in the case of reflection measurement, they are arranged on the same side of the tissue.

The measured intensity can be used to calculate oxygen saturation in the arterial blood of the patient it measured at least at two wavelengths. The mathematical background therefor, which makes use of Lambert-Beer's law, has been described in sufficient detail in a multiplicity of former publications. See, for example, EP-A-262 778 which contains a rather good breakdown of the theory.

Usually, the sensor detachably connected to the pulse oximeter comprises at least two LEDs emitting light of a wavelength of e.g. 660 nm (Nanometers) - red - and 900 nm - infrared. The intensity of the emitted light can be modulated by the oximeter in that the exciting current of the LEDs is varied. The photocurrent received by the receiving element is measured by the oximeter and used to calculate the oxygen saturation of the arterial blood.

In today's oximeters, a hardware circuit contained in the front-end portion of an appropriate monitor makes use of a time multiplex approach, where the LEDs are switched on and off one after the other (see US 4,407,290; US 4,523,279 or US 3,847,483). The train of pulses usually consists of a minimum of 3 phases: an active red, an active infrared and a dark phase, where the ambient light is measured during the latter phase. Actually, there can be more than 3 phases to allow for more LEDs to be powered in one multiplexing time frame or additional dark phases. The phases are often of similar duration. The modulation frequency (repetition rate of the whole frame) ranges e.g. from 200 Hz to 2 kHz. This frequency should not be mixed up with the frequency of the light emitted by the LED's. In fact, the common modulation method uses pulse trains with pulses of rectangular shape to excite the LEDs, wherein the first pulse excites the red LED, the second pulse the infrared LED, and an interval without excitation is used to measure the ambient light (called "dark phase" in this context).

It has turned out, however, that such known oximeters, although widely used today in clinical practice (simply because they offer a non-invasive technology), provide limited performance only and, in particular, limited measurement accuracy. This happens mainly when sources of interference, such as neon lamps, UV lamps and other emitters of light, influence the optical path between the LEDs and the photoreceiver. The susceptibility of prior art oximeters to such sources of interference has been known by skilled people; however, no attempts have been made so far to evaluate the reasons therefor. In fact, the following considerations (which explain the mechanism of distortion of oximetry signals) reflect already the inventors thoughts on this problem, which in turn led him to the present invention, and can insofar already be considered as forming a part of, or - to some extent - the basis of the invention.

The frequency spectrum of a time multiplex signal (as described above) at the receiving photo diode consists of a couple of elements. The first is, of course, the spectral line of the LED modulation frequency. Other spectral lines of reduced amplitude appear at the harmonics of the basic modulation frequency, due to the fact that the spectrum of a pulse sequence contains spectral lines at multiples of the pulse repetition frequency. Such harmonics of significant amplitude appear up to an order of several tens.

Further, all spectral lines are not sharp lines, but rather broadened to some extent, such that they cover small bands in the spectrum. This is due to the fact that the signals are further modulated by the variation of the blood pulse (which contains components up to approx. 10 Hertz). This variation of the blood pulse is actually of interest for medical monitoring. Its frequency may be used to derive the patient's heart rate, and its amplitude is actually required to calculate oxygen saturation.

The blood pulse therefore broadens the spectral lines of the modulation frequency and its harmonics for about ± 10 Hertz. This is also called "physiological bandwidth", due to its origin in the physiological signal.

The details and drawbacks of the prior art technology will now be discussed with reference to the drawings.

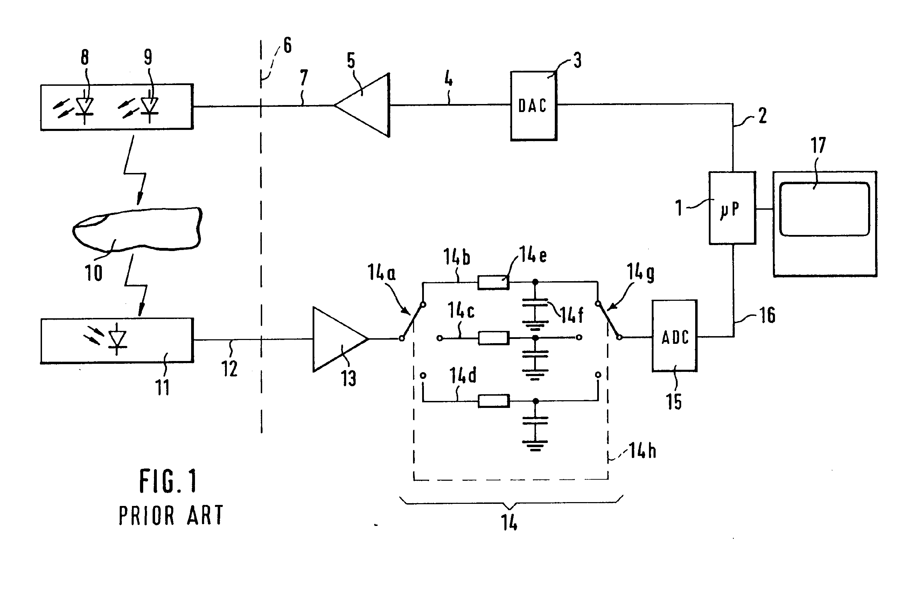

Fig. 1 depicts the essential functional blocks of a prior art oximeter. A microprocessor 1 controls operation of the oxygen saturation parameter. It generates digitally represented pulses for excitation of the LEDs. These are fed, via line 2, to digital-to-analog converter 3 which outputs analog signals of rectangular shape and feeds them, via line 4, to amplifier 5.

Dotted line 6 represents schematically the interface between the monitor and the sensor. That is, line 7 is physically a cable which connects the sensor and the monitor, and all elements left to dotted line 7 are in practice incorporated into the sensor. The sensor contains at least 2 LEDs 8 and 9 which emit light into the tissue of a patient, here a finger 10. Light transmitted through finger 10 reaches photodiode 11 and is fed, via line 12, to another amplifier 13. The amplified analog signal produced by amplifier 13 is fed (line 14) to a demodulation and fitter section 14. This demodulation section comprises a demultiplexer 14a which feeds the amplified signal, depending on its time slot, to three different paths 14b to 14d which implement a low-pass/sample and hold function each. Demultiplexer 14a is controlled such that signals received during operation of LED 8 are fed to path 14b, signals received during operation of LED 9 are fed to path 14c, and signals received during the dark or ambient phase (LED's 8 and 9 switched off) are fed to path 14d.

Each of paths 14b to 14d operates as an independent low-pass filter - see, e.g., resistor 14e and capacitor 14f in path 14b -; the capacitor acts insofar also as a sample and hold device. The three paths 14b to 14d are then combined again by multiplexer 14g (which is synchronized with demultiplexer 14a, see dotted line 14h), and the signals are fed to analog-to-digital converter (ADC) 15. This ADC samples the incoming signals, typically once per channel, but a higher sampling rate is also possible. In other words, if a pulse is sent to the red LED 8, it is sensed once by ADC 15 to determine its amplitude, and the variation of the amplitude in succeeding pulse trains reflects pulsatile variation of the arterial blood.

The digitized signal reaches, via line 16, microprocessor 1 which performs the necessary calculations to determine oxygen saturation. The results are then displayed on a display 17.

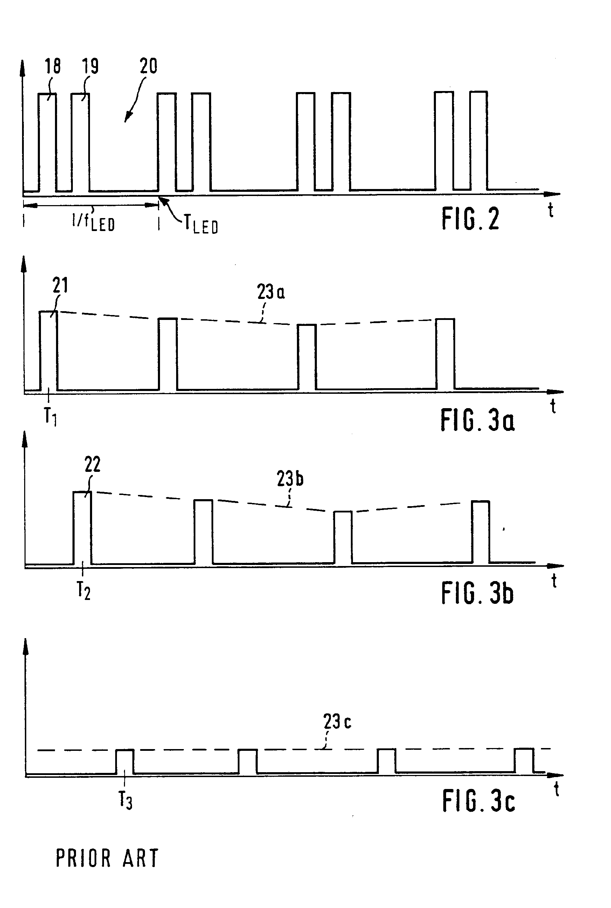

Fig. 2 is a timing diagram of the pulse train used to excite LEDs 8 and 9. A first pulse 18 controls red LED 8 (Fig. 1), and a second pulse 19 infrared LED 9. When both LEDs have been excited in sequence, a dark phase 20 is provided which is used on the receiver side to measure the amount of ambient light. At t=$T_{LED}$ , the whole pattern starts again from the beginning. Thus, the modulation frequency is defined by

$$f_{LED} = \frac{1}{T_{LED}} \tag{1}$$

Figs. 3a to 3c depict the same pattern at the receiver side, separated according to paths 14b to 14d (Fig. 1), i.e., when the pulses have passed the human tissue. Fig. 3a shows the pulse of the red LED 8 (path 14b), Fig. 3b the pulse of the infrared LED 9 (path 14c), and Fig. 3c the signal detected on path 14d (ambient light). One will note that the red and infrared pulses (Figs. 3a and 3b) are

1. attenuated by a more or less fixed amount which is caused by non-pulsating tissue (DC attenuation);

2. subject to different attenuation in the red and infrared channel, as shown by the different amplitudes of red pulse 21 and infrared pulse 22; and that

3. their amplitudes are modulated with a slow frequency shown by dotted lines 23a and 23b (the frequency is even exaggerated in Figs. 3a and 3b for the purpose of demonstration), i.e., their amplitudes vary slowly over time. This slow frequency - in the range of 1 to 10 Hertz - reflects the blood pulsation in blood vessels hit by the light emitted by LEDs 8 and 9 and carries the physiological information required to calculate oxygen saturation. It is therefore called "physiological signal", and the associated frequency band "physiological bandwidth". It will also be noted that the superimposed physiological signal has a very small amplitude, as compared to the overall amplitude of the received pulses.

In contrast, the pulse of Fig. 3c which represents ambient light is not modulated by pulsating tissue (ref. no.

23c).

The pulse trains shown in Figs. 3a to 3c are fed, via multiplexer 149 (Fig. 1), to the input of ADC 15. According to the prior art approach, ADC 15 takes at least one sample of the received pulse at $t=T_1$, $t=T_2$ and $t=T_3$. These samples represent the amplitudes of the red pulse, the infrared pulse and the ambient light, respectively. The technique discussed here corresponds to a demodulation with a rectangular or square wave.

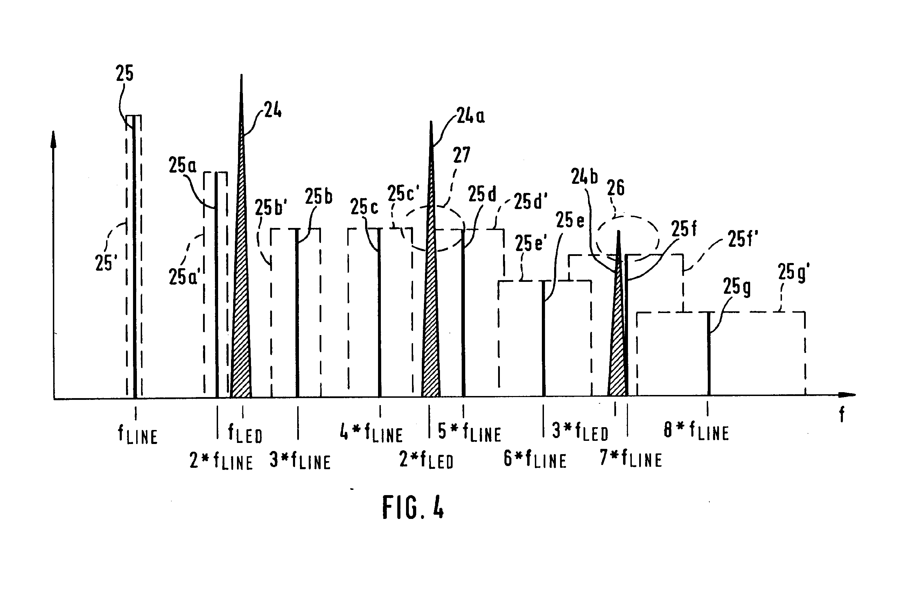

Now let us consider what happens in the frequency domain. Fig. 4 depicts the spectrum of the signal transmitted through the patient's tissue and amplified, but prior to demodulation and sampling, i.e., as it appears on line 14 (Fig. 1). The basic LED modulation frequency $f_{LED}$ is shown as spectral line 24. This is not a "sharp" spectral line. Instead, it is slightly broadened, due to the physiological signal (as discussed above) which in turn modulates the LED signal. The effect is that the LED modulation spectral line appears in fact as a small-band spectrum with a bandwidth of approx. ± 10 Hertz around the center of its base frequency.

Reference numbers 24a and 24b designate harmonics of the basic LED modulation freqency, i.e., $2*f_{LED}$ and $3*f_{LED}$. It is understood that these harmonics appear also broadened by the physiological bandwidth, i.e., they are centered at ± 10 Hertz around the corresponding harmonic frequency.

In contrast, reference number 25 represents the spectral line of the mains (power line) - at $f_{Line}$ - which is the major source of interference; typically, at 50 Hertz (Europe) or 60 Hertz (United States). It is understood that, although the power line has always a specific frequency, this frequency is subject to variations (however small and slow) of the mains frequency. This variability or tolerance band is indicated by dashed box 25'.

The spectrum also contains harmonics of the basic mains frequency $f_{Line}$. These are denoted as 25a through 25g in Fig. 4 and represent frequencies of $2*f_{Line}$ to $8*f_{Line}$ (it will be appreciated that harmonics of even higher order do also exist, but have not been drawn in Fig. 4). Like the basic mains frequency, their harmonics are also spread, see dotted boxes 25a' through 25g'. The diagram shows that the higher the order of the harmonics, the higher the possible variations in frequency.

Fig. 4 reveals also another effect. That is, some of the harmonics of the LED modulation frequency $f_{LED}$, and of the mains frequency $f_{Line}$, are quite close to each other. A typical example is shown by reference number 26. The third order harmonics 24b of the LED modulation frequency, and the seventh order harmonics 25f of the mains frequency, are quite close to each other ($3*f_{LED} \approx 7*f_{Line}$). This means that there is frequent interference in the related bands, in particular in consideration of the broad tolerance band 25f' associated with the seventh order harmonics 25f of the mains frequency. But there are also other doubtful cases, such as indicated by dotted circle 27. Although there is in fact some distance between the second order harmonics 24a of the LED modulation frequency and the fifth order harmonics 25d of the mains frequency, there is still an overlap if we consider the whole tolerance band 25d' associated with $5*f_{Line}$. In other words, if the frequency of the power line deviates slightly from its nominal value, its fifth order harmonic might approach the second order harmonic of the LED modulation frequency, such that there is at least sporadic interference. If we proceed to higher order harmonics, there will always be a danger of interference, just because the tolerance band associated with the harmonics of the mains frequency becomes broader and broader, such that the associated spectra overlap. This is shown by spectra 25e', 25f' and 25g'. It is evident that for these higher frequencies, any harmonic of the LED medulation frequency will necessarily fall into at least one bandwidth of the harmonics of the mains frequency.

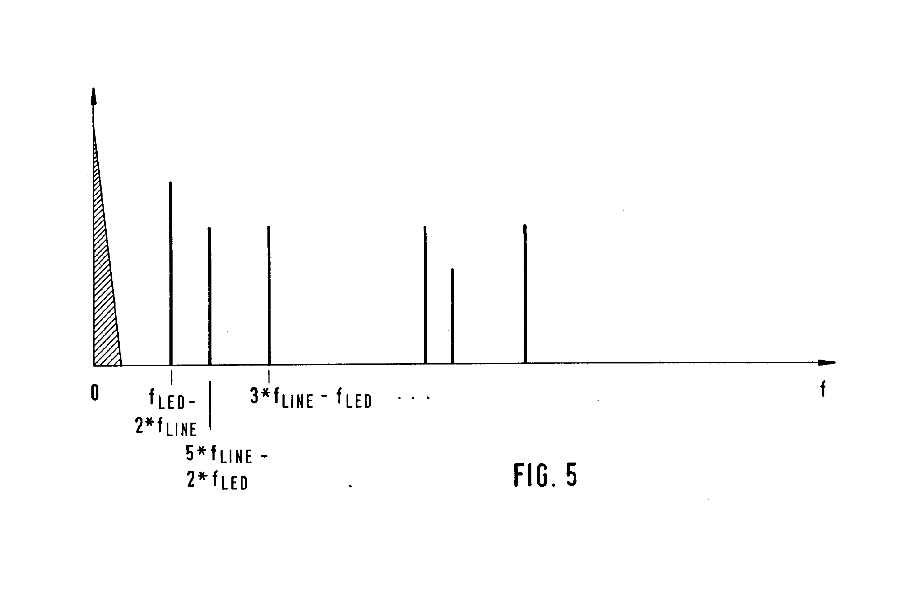

One might now ask the question how such interference of the harmonics influences the base band and thus the results of oxygen saturation measurement. The underlying mechanism is demonstrated in Fig. 5.

According to the prior art approach, the received signal is demodulated by demultiplexer 14a. (This kind of demodulation corresponds to synchronous AM demodulation with a square wave, as e.g. described in US 4,807,630). Demodulation with a square wave has the effect that all kinds of difference and sum frequencies-in particular, of the harmonics - appear in the base band close to the signal of interest; i.e., the harmonics are folded down into the base band. This effect is, by way of example, illustrated in Fig. 5. This figure is based on the understanding that the LED sampling frequency is equal to the LED modulation frequency. (Every channel - red, infrared, dark - is separately sampled and demodulated).

However, the harmonics of the mains frequency are only one source of interference which may distort the useful signal for determining oxygen saturation. In a clinical environment, the pulse oximetry sensor picks up ambient light and various electromagnetic noise. The major source for ambient light is room illumination with fluorescence ceiling lamps which gives broad spectral bands with harmonics at harmonics of the power line frequencies, typically 50 Hertz or 60 Hertz. However, electrical noise also comes very often from the power line and shows up as harmonics of the mains frequency. Other well known sources for largely interfering electrical noise are electro-surgery devices used in the operating rooms. They can be very broad-band and at any frequency.

As explained above, the spectra of the signal at multiples of the LED modulation overlap very likely with the spectra of the optical or electrical noise components. Any noise lines in one of the LED modulation bands will be demodulated and intrinsically folded down to the base band and contribute to poor signal-to-noise ratios (S/N). A very dangerous situation for the patient can occur in the monitoring of neonates. These are often treated with very bright UV lamps for the bilirubin photo therapy. As they produce poor signals because of a poor vascular perfusion, the amount of ambient light can cause even situations with a signal-to-noise ratio < 1. A pulse oximeter is very likely to be mislead in these situations. It can derive values for pulse rate, oxygen saturation and perfusion index which are wrong because the input signals are dominated by noise instead of patient signals.

EP0502717 A1 discloses means and methods for evaluating the concentration of a constituent in an object by measuring the transmission of light of two wavelengths therethrough. First and second light emitters emit light at respective, first and second different wavelengths. A modulator/driver drives the light emitters with respective first and second carriers which vary as a function of time, the carriers being of the same carrier frequency having a phase difference other than 0 and other than an integer multiple of 180°. A detector receives light from the first and second light emitters after the same has passed through the object and generates a resulting detector signal carrying information relating to transmission of the object at both wavelengths. A first demodulated signal which is a sum of a component proportional to the object's transmission at the first wavelength and one or more carrier modulated components is generated by a demodulator from the detector signal in a first channel, whereas a second demodulated signal which is a sum of a component proportional to the object's transmission at the second wavelength and one or more carrier modulated components is generated by the demodulator in a second channel. The carrier modulated components of the signals are filtered out of the first and second channels by a demodulated signal filter.

The demodulator multiplies the detector signal in the first channel with a sinusoidal signal in phase with the first carrier to generate the first demodulated signal, and multiplies the detector signal in the second channel with a sinusoidal signal in phase with the second carrier to generate the second demodulated signal. Therefore, in accordance with the disclosure of EP 0502717 A1, the sinusoidal signal for demodulating the detector signals in the first and the second channel are in phase with the first carrier and the second carrier, respectively. This demodulation method is disadvantageously to the effect that there exists a cross-talking between the first and the second channels, and therefore no accurate evaluation of the concentration of a constituent in an object is possible.

EP-A-0102816 relates to a pulse oximeter wherein light of two different wavelengths is passed through human or animal body tissue. The level of incident light is continually adjusted for optimal detection of the pulsatile component, while permitting accommodation to variable attenuations due to skin color, flesh thickness and other invariants.

US-A-4800885 describes an apparatus and a method for measuring the level of a constituent such as oxygen in the blood of a living subject. Light at a plurality of wavelengths is emitted and directed to through the patient's body to a photodetector. The amplitude of the emitted light at each wavelength is varied in accordance with a different carrier frequency, and the photodetector signal thus includes a component at each carrier frequency. The photodetector signal is subdivided by frequency so as to separate the components at the different carrier frequencies. The constituent level is determined from these separated components.

WO-A-9213598 relates to time and frequency domain spectroscopy for determination of the saturation of a tissue region. This document teaches determining saturation readings from phase shifts of signals transmitted through a tissue region. Alternatively, the real and the imaginary portions of the received signal are separated, the obtained data being used along with the data gathered from a DC portion of the signal to determine saturation.

Starting from this prior art, it is the object of the present invention to provide methods and apparatus for accurately measuring and analysing medical parameters using at least two different electromagnetic waves having different wavelengths, wherein a cross-talking between channels in which received electromagnetic waves of different wavelength are analysed is reduced.

This object is achieved by methods for measuring medical parameters in accordance with claims 1,3 and 8 and apparatus for measuring medical parameters in accordance with claims 19, 21 and 26 of the present application.

The present invention is based on the perception that in prior art analysis systems for measuring medical parameters, for example the system that is disclosed in EP 0502717 A1, an accurate measurement and subsequent analysis of medical parameters is impossible due to a phase shift which is introduced by the system between the electromagnetic waves which are used to irradiate a sample and the electromagnetic waves which are used for analysis. Therefore, the present invention provides methods and apparatus for measuring medical parameters of a patient by irradiation of electromagnetic waves into a sample and for measurement and subsequent analysis of the electromagnetic waves which have passed through said sample, wherein the system phase shift is compensated during the analysis of the received electromagnetic waves.

In accordance with a first aspect of the present invention according to claim 1, the first and second sinusoidal demodulation signals comprise a phase difference relative to the first and second modulation signals which corresponds to the system phase shift, whereby the influence of the system phase shift on the result of the analysis is eliminated and therefore a cross-talking between channels is reduced.

In accordance with a second aspect of the present invention according to claim 3, demodulating signals of received electromagnetic waves are demodulated by sinusoidal demodulation signals to generate a first and a second demodulated signal. These first and second demodulated signals are analysed, for example by a microprocessor, under consideration of a system phase shift. On the basis of this system phase shift a correction matrix is calculated which is used to correct the demodulated signals in order to eliminate the influence of the system phase shift on the result of the analysis. In accordance with this second aspect of the present invention, the influence of the system phase shift on the result of the analysis can be eliminated by software, for example in the central processing unit of the system.

In accordance with a third aspect of the present invention according to claim 8, the influence of the system phase shift on the result of the analysis is eliminated by adding a phase shift to the received electromagnetic waves, such that a total phase shift of the received electromagnetic waves relative to transmitted electromagnetic waves is substantially an integral multiple of 360°. This total phase shift of an integral multiple of 360° makes sure that a cross-talking between channels of the received electromagnetic waves is reduced.

In accordance with a preferred embodiment of the present invention, the frequency of the modulation signals is determined to be approximately f=275 Hertz. This selection guarantees that the main spectral line of the modulation frequency is placed optimally between harmonics of the noise bands for 50 Hz and 60 Hz mains frequency, even if there is a variation or instability in the mains frequency (see detailed description). Therefore, this selection of the modulation frequency further contributes to a noise-free, reliable measurement.

The modulation signals themselves may be sine waves, in which case the mathematical theory becomes particularly easy. However, square or rectangular waves may be used as well. Square waves are easy to generate and have another related advantage: That is, the duty cycle may be easily varied without changing the fundamental frequency, in order to adapt the signal strength in various channels. (The shape of the modulation signals should not be mixed up with the signals used for demodulation; according to the invention, the latter have always to be sinusoidal signals).

In a preferred embodiment of the invention, a low pass filter is provided through which the output signal of the multiplier circuit is fed. Likewise, it is advantageous to use a bandfilter connected between the receiver of the electromagnetic waves and the demodulator. This bandfilter blocks the harmonics, and even the fundamental frequencies of any noise, and further operates as an anti-aliasing filter for subsequent analog-to-digital conversion.

As already mentioned, one major application of the invention is pulse oximetry. In this case, the biological material examined *in vivo* is the patient's tissue. The electromagnetic waves are preferably waves selected from the visible and the adjoining spectra of light, in particular red and/or infrared light. The emitters of light are advantageously light-emitting diodes which are of small size and easy to incorporate into a sensor.

Embodiments and developments of the present invention are defined in the dependent claims.

Preferred embodiments of the present invention will now be explained, by means of non-limiting examples, with reference to the accompanying drawings, in which:

| | |
|---|---|
| Fig. 1 | depicts a block diagram of a prior art oximeter, |
| Fig. 2 | is a timing diagram of the pulse train emitted by a prior art oximeter, |
| Figs. 3a to 3c | are timing diagrams of the pulse trains received by a prior art oximeter, |
| Fig. 4 | is the spectrum of a prior art time multiplex oximeter prior to demodulation, |
| Fig. 5 | depicts the effect of demodulation on the spectrum in prior art oximeters, |
| Fig. 6 | is a block diagram of a pulse oximeter according to the present invention, |
| Fig. 7 | depicts the shape and timing of the pulses used to control the light-emitting diodes, |
| Fig. 8 | is a block diagramm of the digital signal processor, |
| Fig. 9 | is an example of a spectrum processed by an apparatus according to the present invention, |
| Fig. 10 | shows the effect of the quadrature modulation technique according to the present invention on the signal-to-noise improvements, |
| Fig. 11 and Fig. 12 | are vector diagrams for illustrating phase shift measurements, and |
| Fig. 13 | is a schematic representation for illustrating direct phase measurement on an unfiltered signal. |

Figs. 1 through 5 illustrating the prior art approach have already been discussed above, such that there is no need to consider them further.

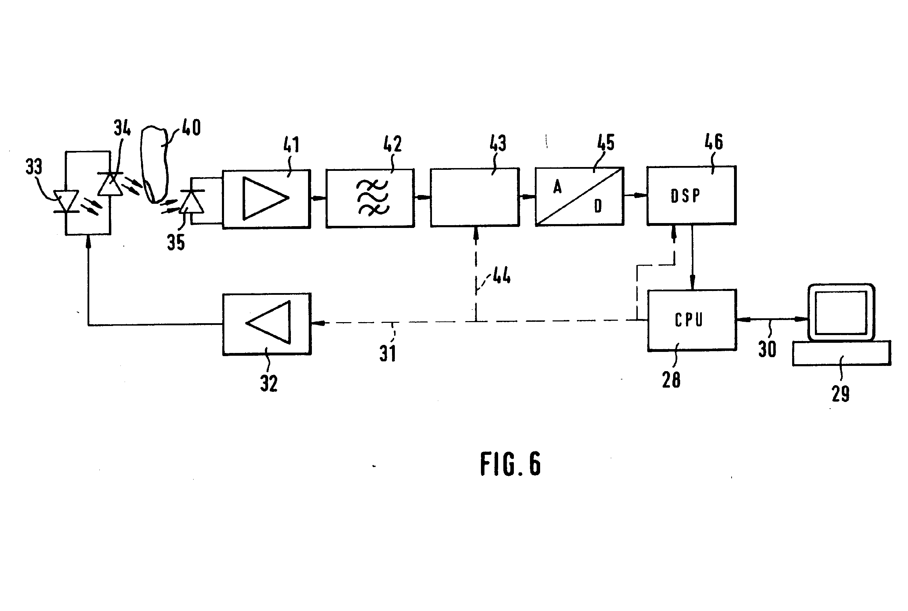

Fig. 6 depicts a block diagram of a two-wavelength pulse oximeter according to the invention. A microcontroller or CPU (central processing unit) 28 controls overall operation of the oximeter. In particular, it starts the measurements, adjusts the gain and controls the timing of LED excitation. Further, most of the signal processing, in particular calculation of the pulse oximetry values (oxygen saturation), of the pulse rate, the perfusion indicator and plethysmograhic waveforms is performed by CPU 28. Such algorithms - specifically suited for artifact suppression - are, for example, described in another patent, US 5,299,120, originating from the inventor of the present case. CPU 28 may further communicate with other equipment, such as a host monitor 29, via a digital link 30. The host monitor is equipped with data and waveform display capabilities, controls the alarm limits etc. It is, however, understood that the display of data, manual input etc. could also be performed locally. Further, CPU 28 may even communicate with larger systems such as hospital information systems or central stations.

The control signals for driving, i.e., for switching the LEDs are fed from CPU 28, via line 31, to an LED driver circuit 32 (the digital-to-analog converter is not shown here and may be integrated in CPU 28). LED driver circuit 32 contains current sources which operate light emitting diodes (LEDs) 33 and 34 in switched mode.

In Fig. 6, a red LED is designated as 33, and an infrared LED as 34. Both LEDs, as well as a photodiode 35, are integrated into an appropriate sensor. Such sensors are well-known in the art and need not be discussed in detail here. Reference is made, for example, to DE-C-37 03 458. The rest of the elements shown in Fig. 6 are not incorporated in the sensor, but in an appropriate monitor, a signal pick-up box or the like.

LED driver circuit 32 operates LEDs 33 and 34 in antiparallel mode. That is, red LED 33 is operated by pulses of one polarity, and infrared LED 34 is operated by pulses of the opposite polarity. This design requires less connections between the sensor and the associated monitor, although it is not a necessary prerequisite for practising the present invention,

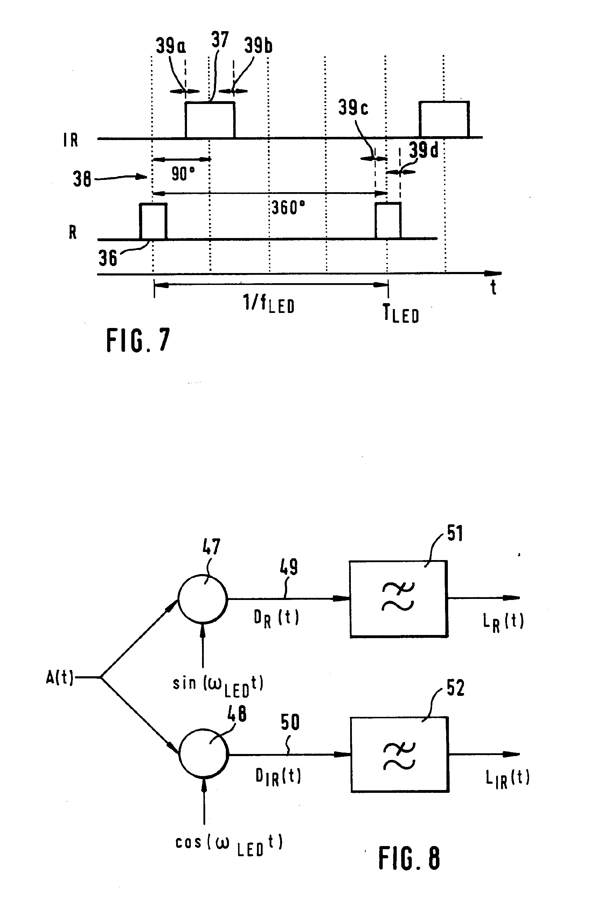

The timing of the pulses generated by LED driver circuit 32 will now be discussed with reference to Fig. 7. In the shown example, these pulses are of rectangular shape, although they could also be shaped as a sine, or incorporate any other suitable shape.

The pulse driving the red LED 33 is designated as 36. Another pulse 37 (of opposite polarity, not shown in Fig. 7) drives infrared LED 34. The whole pattern restarts at $t=T_{LED}$, such that a time interval of $T_{LED}$ defines a complete cycle of 360°. An important design feature of the timing chosen in the pulse oximeter according to the invention is that the pulses of the red and the infrared LED, respectively, are offset by 90°, see reference number 38. (In terms of timing, this phase shift corresponds to $T_{LED}/4$). The reference lines for the 90° phase shift are the center of the red and infrared pulse, respectively. This is of importance if a duty cycle adjustment is necessary or desirable to balance the red and infrared signals. Such duty cycle adjustment means to adapt the pulse length of either or both of the pulses, as indicated by arrows 39a to 39d.

Returning now to Fig. 6, the light pulses generated by LEDs 33 and 34 pass human tissue, as indicated by a finger 40, and are received by photodiode 35. The output signal of the photodiode is fed to a photo amplifier 41. This photo amplifier converts the photo current of the photodiode into a voltage. In contrast to prior art time multiplex systems, its bandwidth can be limited to $f_{LED}$ in the present invention. Its gain is selected as high as possible, but within the limits of the maximum expected photo currents.

Reference number 42 designates a band filter. The main purpose of that stage is to block out-of-the band noise and to serve as an anti-aliasing filter for analog-to-digital sampling. The passband is designed as narrow as possible around the center frequency $f_{LED}$. The bandwidth is only slightly broader than the bandwidth of the physiological blood pulse signal. Any harmonics of the LED sampling frequency can be blocked out as they do not contribute to the demodulated signal.

Programmable gain amplifier 43 (controlled by CPU 28, see line 44) ensures that the filtered signal fits within the input range of analog-to-digital converter (ADC) 45. It is therefore used to adapt to the large dynamic variability of the photoelectrical signal As pulse oximetry sensors are used on very different body locations, the different tissue types and thickness between emitters and receivers result in largely different light intensities. The gain of that amplifier is controlled by the CPU to adapt the output amplitude to fit the full scale input range of the analog-to-digital converter for good quantization.

Digital signal processor (DSP) 46 represents, to some extent, the "heart" of the present invention. It demodulates the received, filtered and digitized pulses with sinusoidal functions. In the shown embodiment, this demodulation is performed in the digital domain - i.e., by a digital processor -, and in fact the functionality of DSP 46 may be provided by CPU 28 as well. However, this is not a necessary requirement; DSP 46 may also be a separate signal processor in digital or analog technology. The major functions of DSP 46 are demodulation, low pass filtering and down sampling.

In order to explain the functionality provided by DSP 46, reference is now made to Fig. 8. The output signal of ADC 45, A(t), is fed to two multipliers 47 and 48 which perform a multiplication with sinusoidal functions $\sin(\omega_{LED}t)$ and $\cos(\omega_{LED}t)$, respecitvely. The skilled man will immediately note that the use of sine and cosine functions corresponds to a phase shift of 90°, just because $\sin(90°+\alpha) = \cos(\alpha)$. The demodulated signals are now fed, via lines 49 and 50, to respective digital low pass filters 51 and 52. Their functionality will be discussed in greater detail below.

For the following discussion of the mathematical theory, it will be assumed that the LEDs are driven so that the optical emissions of the LEDs are pure sine waves, shifted by 90° in phase, e.g. the red LED with a sine and the infrared LED with a cosine. In the actual embodiment, this is not the case, as shown above; however, the mathematical background may be explained more easily by the assumption of sinusoidal driving.

The output of ADC 45, i.e.,the input signal to DSP 46 is then

$$A(t) = A_R(t) + A_{IR}(t) \tag{2}$$

with

$$A_R(t) = A_R{}^* \sin(2\pi {}^* f_{LED}{}^* t + \varphi) \tag{3a}$$

$$A_{IR}(t) = A_{IR}{}^*\cos(2\pi{}^*f_{LED}{}^*t+\varphi) \qquad (3b)$$

wherein $\varphi$ is the phase shift between the optical LED signal and the digitized photo receiver signal at the ADC output, herein also referred to as system phase shift, and $\omega_{LED}=2\pi{}^*f_{LED}$.

$A_R$ and $A_{IR}$ themselves are modulated by the patient's blood pulse:

$$A_R = S_R(t) \qquad (4a)$$

$$A_{IR} = S_{IR}(t) \qquad (4b)$$

wherein $S_R(t)$ and $S_{IR}(t)$ are composed of a DC and an AC component. (The DC component represents the constant tissue absorption, whereas the AC component is related to the variable absorbance due to pulsatile blood volume change).

Demodulation as multiplication with sine waves reveals then at the outputs of multipliers 47 and 48:

$$D_R(t) = A(t){}^*M_R(t) \qquad (5a)$$

$$D_{IR}(t) = A(t){}^*M_{IR}(t) \qquad (5b)$$

with

$$M_R(t) = \sin(\omega_{LED}{}^*t+\sigma) \qquad (5c)$$

and

$$D_{IR}(t) = \cos(\omega_{LED}{}^*t+\sigma) \qquad (5d)$$

As will be explained below in more detail, the CPU is programmed to determine the system phase shift $\varphi$ in order to make the demodulator phase shift $\sigma$ equal to the system phase shift $\varphi$.

Given that the demodulator phase $\sigma$ is made equal to the system phase shift $\varphi$ ($\sigma = \varphi$) and solving the equation results in the following fractions:

$$D_R(t) = \tfrac{1}{2}A_R - \tfrac{1}{2}A_R{}^*\cos(2\omega_{LED}{}^*t+2\varphi) + \tfrac{1}{2}A_{IR}{}^*\sin(2\omega_{LED}{}^*t+2\varphi) \qquad (6a)$$

$$D_{IR}(t) = \tfrac{1}{2}A_{IR} - \tfrac{1}{2}A_{IR}{}^*\cos(2\omega_{LED}{}^*t+2\varphi) + \tfrac{1}{2}A_R{}^*\sin(2\omega_{LED}{}^*t+2\varphi) \qquad (6b)$$

So far, the demodulated signals contain both the sum and difference frequencies. These signals are then fed through digital low pass filters 51 and 52 which have a cut-off frequency just beyond the physiological bandwidth of the blood pulse. In an ideal low pass filter, this operation cuts off all harmonics of the modulation frequency $f_{LED}$.

In this case, the output of low-pass filters 51 and 52 is

$$L_R(t) = \tfrac{1}{2} A_R \qquad (7a)$$

$$L_{IR}(t) = \tfrac{1}{2} A_{IR} \qquad (7b)$$

These signals correspond ideally to the optical absorption of the red and infrared signals at the photodiode. Note that signals $A_R$ and $A_{IR}$ are not constant, but are modulated by the blood pulsation itself.

Signals $L_R(t)$ and $L_{IR}(t)$ are now small-band signals, which means that the data rate is reduced to a level desirable by standard down-sampling techniques. It is further understood that all other frequencies in the received signal, like electrical or optical interference, are blocked completely by the method according to the present invention. The result is very pure, noise-free signal reconstruction. Therefore, it is possible to choose an interference-free modulation band, even at lowest frequencies. In the preferred embodiment of the present invention, the LED modulation frequency is selected as $f_{LED}$ = 275 Hertz.

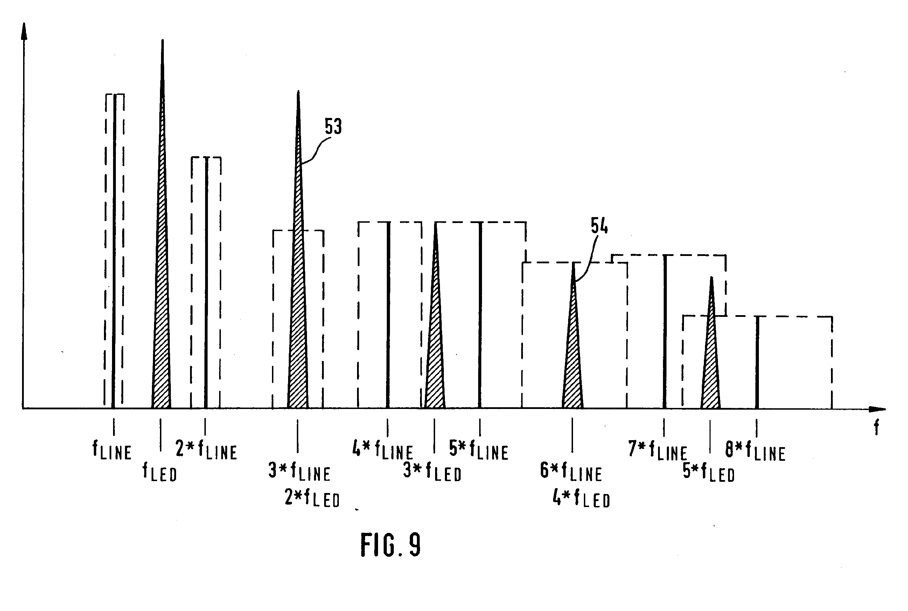

In order to explain the advantages of the present invention, as compared to the prior art time multiplex approach, and in particular the advantages of demodulation with sine waves, preferably with a phase offset of 90°, reference is now made to Fig. 9. This figures depicts a typical spectrum in a representation similar to Fig. 4. However, Fig. 9 depicts even a more sensitive case, just because some of the harmonics of the LED modulation frequency, and some harmonics of the mains frequency coincide. Reference numbers 53 and 54 relate to such cases:

$$2^*f_{LED} \approx 3^*f_{Line} \text{ (Ref. no. 53)} \qquad (8a)$$

$$4^*f_{LED} \approx 6^*f_{Line} \text{ (Ref. no. 54)} \qquad (8b)$$

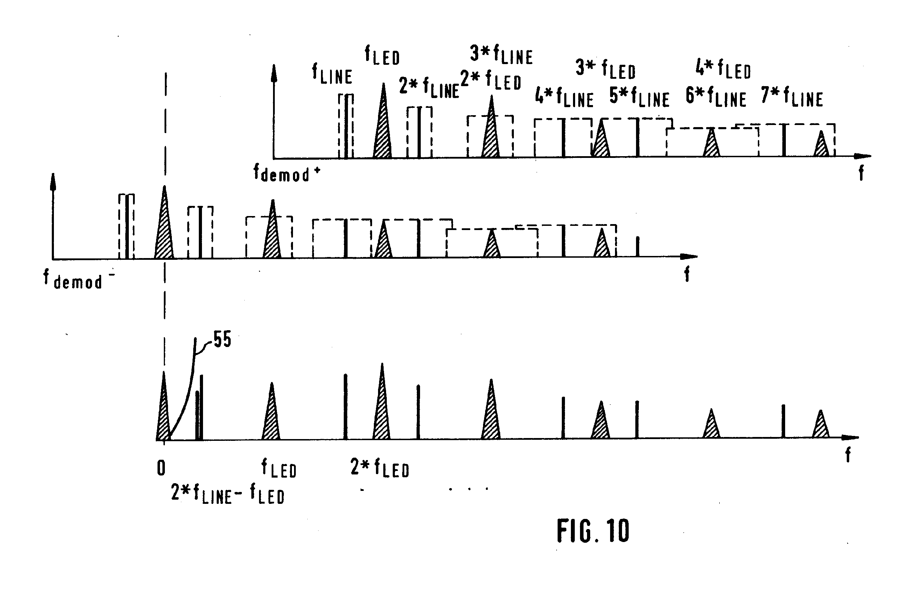

The demodulation technique according to the present invention is, however, still able to filter the harmonics of the mains frequency out, as will now be shown by means of Fig. 10.

The quadrature demodulation technique according to the present invention, in combination with sampling, produces a shift of the related spectra, together with the generation of sum and difference frequencies. The amount of frequency shift is $f_{demod}$, i.e., the frequency of the sine functions used for demodulation, which is in this case identical to the LED modulation frequency $f_{LED}$. The shifted spectra (two in total) are shown in Fig. 10, namely the spectrum shifted by $+f_{demod}$ (upper diagram) and the spectrum shifted by $-f_{demod}$ (second diagram from top). (The frequency designations are, except of the shift, identical in both diagrams, so they are only shown in the upper one).

The resulting spectrum is shown in the lower diagram. A low pass filters out the base band and blocks all higher frequencies (its attenuation characteristics is designated by reference number 55). As can be easily observed, the baseband signal does not contain any noise resulting from the harmonics of the power line or other sources of interference, which makes up the great advantage of the present invention over the prior art approach.

What follows is a detailed description of preferred embodiments of the present invention which are useful to eliminate the influence of the system phase shift $\varphi$ out of the result of the analysis.

In accordance with the first embodiment, the system phase shift $\varphi$ of the system, shown for example in Fig. 6, is determined, and on the basis of the determined system phase shift $\varphi$ the phase shift $\sigma$ of the sinusoidal demodulation signals with respect to the first and second modulation signals is adjusted.

An important function is the capability to measure the phase shift $\varphi$ of the system. This phase shift $\varphi$ is defined as the phase difference between the excitation signals which drive each LED and the receiving signal as sampled by the A/D converter.

A software routine in the CPU performs that measurement routinely at power up and selected events during normal operation. Events can be programmed time intervals, a change of the sensor connected, changing the gain of programmable gain amplifier, and thereby its filter characteristics, and so on.

Subsequently, a particular embodiment of a procedure for determining the system phase shift $\varphi$ is described.

Firstly, the normal operating mode is interrupted. The red LED is disabled, whereas the infrared LED is driven in its normal mode. Then, in the preferred embodiment, a default phase a equal $\sigma_{DEF}$ is applied to the demodulator. After a waiting time of some milliseconds, until the low pass filters have settled, the real-time values in the red channel $L_R'$ and the infrared channel $L_{IR}'$ are stored. On the basis of this stored real-time values the correct phase shift $\sigma$ to be applied to the demodulator is calculated, see the following equation. Afterwards, the correct phase shift $\sigma$ is applied to the demodulator.

Subsequently, the red LED is enabled again. After having awaited until low pass filters have settled again, the normal operating mode is resumed.

The calculation of the correct phase shift a is done by the following equation:

$$\sigma = \sigma_{DEF} + \in \qquad (13)$$

with

$$\varepsilon = \arctan\left(\frac{L_R'}{L_{IR}'}\right)$$

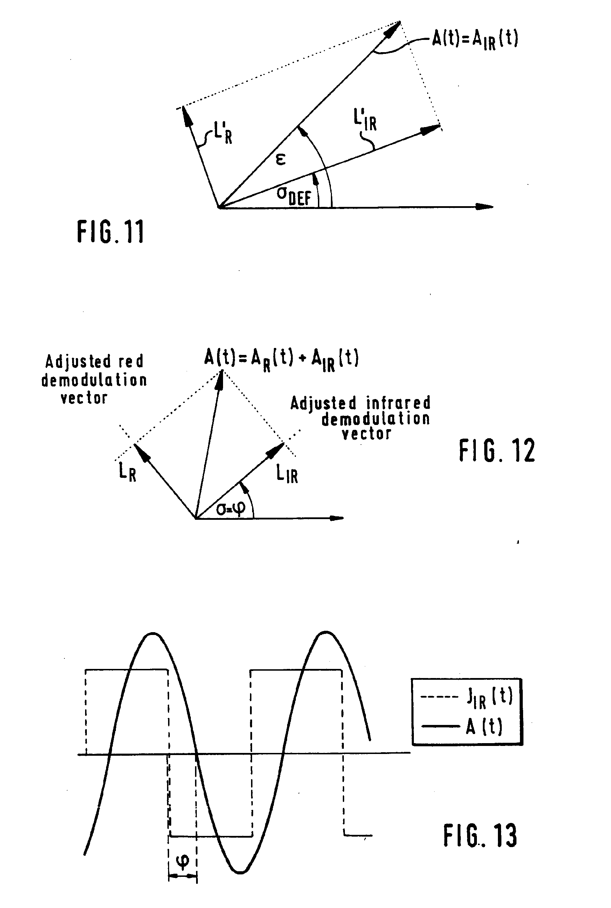

The vector diagram shown in Fig. 11 shows the situation during the phase measurement. The arrow designated by $L_R'$ represents the red demodulation vector. The arrow designated by $L_{IR}'$ represents the infrared demodulation vector. The arrow arranged between these arrows represents the received electromagnetic wave A(t). It is: $A(t) = A_{IR}(t)$ since $A_R(t) = 0$ during phase measurement. This phase adjust vector diagram represents the situation prior to adjustment.

The value of the demodulated signal in the red channel would be close to 0, if the default phase ($\sigma_{DEF}$) was already correct. In general, a cross-talk $L_R'$ from the infrared exitation exists because the default phase (or the phase from a prior adjustment) is only close to the correct value, that means the correction angle $\varepsilon \neq 0$.

The vector diagram shown in Fig. 12 shows the situation after having applied the phase adjustment. The arrow on the left hand side $L_R$ represents the adjusted red demodulation vector. The arrow on the right hand side $L_{IR}$ represents the adjusted infrared demodulation vector. The middle arrow represents the received electromagnetic waves A(t) being a combination of the electromagnetic wave $A_R(t)$ (red excitation) and the electromagnetic wave $A_{IR}(t)$ (infrared excitation). As can be seen from Fig. 12, the correct phase shift $\sigma$ to be applied to the demodulator corresponds to the system phase shift $\varphi$.

The advantage of this auto-phase-adjust is that it compensates for any phase shifts and drifts which can occur for reason of temperature, aging and component tolerances. It also adapts to changing sensor characteristics. A further advantage of this implementation of automatic adjust is that it can be applied without interruption of the continuous plethysmographic wave form, if the pleth signal is derived from the infrared LED.

It is understood that the role of red and infrared could be exchanged without changing the fundamental idea.

Another advantage of adjusting the demodulation phase to match the system phase, $\sigma = \varphi$, is that the demodulated and filtered signal $L_R$ and $L_{IR}$ are only positive. This avoids restricting the numeric range by a factor of two in order to allow for signed integer values.

What follows is a preferred embodiment of a further method to compensate for the system phase shift $\varphi$ in accordance with the present invention. The method described below determines the system phase too as explained above, but does not apply a compensation afterwards. It rather uses the determined or measured phase $\varphi$ in a correction calculation.

In the beginning, the demodulator is running with any fixed phase. In the example below it is set to $\sigma = \sigma_{DEF} = 0$ for simplification of the equations.

Using equations (1) to (5d) yields:

$$D_R(t) = \tfrac{1}{2}A_R[\cos(\varphi) - \cos(2\omega_{LED}{}^*t+\sigma)] + \tfrac{1}{2}A_{IR}[\sin(-\varphi){}^*\sin(2\omega_{LED}{}^*t+\sigma)] \qquad (14a)$$

$$D_{IR}(t) = \tfrac{1}{2}A_R[\sin(\varphi) + \sin(2\omega_{LED}{}^*t+\sigma)] + \tfrac{1}{2}A_{IR}[\cos(\varphi){}^*\cos(2\omega_{LED}{}^*t+\sigma)] \qquad (15a)$$

Setting $\sigma = 0$ yields:

$$D_{IR}(t) = \tfrac{1}{2}A_R[\cos(\varphi) - \cos(2\omega_{LED}{}^*t)] + \tfrac{1}{2}A_{IR}[\sin(-\varphi){}^*\sin(2\omega_{LED}{}^*t)] \qquad (14b)$$

$$D_{IR}(t) = \tfrac{1}{2}A_R[\sin(\varphi) + \sin(2\omega_{LED}{}^*t)] + \tfrac{1}{2}A_{IR}[\cos(\varphi){}^*\cos(2\omega_{LED}{}^*t)] \qquad (15b)$$

After low-pass filtering the output signals are:

$$L_R(t) = \tfrac{1}{2}A_R {}^* \cos(\varphi) + \tfrac{1}{2}A_{IR} {}^* \sin(-\varphi) \qquad (16)$$

$$L_{IR}(t) = \tfrac{1}{2}A_R {}^* \sin(\varphi) + \tfrac{1}{2}A_{IR} {}^* \cos(\varphi) \qquad (17)$$

On the basis of equation (16) and equation (17) a matrix can be defined:

$$[C] = \begin{bmatrix} \cos\varphi & -\sin\varphi \\ \sin\varphi & \cos\varphi \end{bmatrix} \qquad (18)$$

The electromagnetic waves received by photodiode 35 can be written as an input vector:

$$[A] = \begin{bmatrix} A_R \\ A_{IR} \end{bmatrix} \qquad (19)$$

After the demodulation by DSP 46 an output vector can be defined:

$$[L] = \begin{bmatrix} L_R \\ L_{IR} \end{bmatrix} \qquad (20)$$

On the basis of the matrix [C], the input vector [A], and the output vector [L], the original signals ($A_R$ and $A_{IR}$) can be recovered continuously by computing the matrix correction:

$$[A] = [C]^{-1} \cdot [L] \tag{21}$$

$[C]^{-1}$ is the inverse matrix of $[C]$.

The phase measurement procedure is described below.

Firstly, the normal operating mode is interrupted, the red LED is disabled and the infrared LED is continuously driven in normal mode. After waiting for some milliseconds until the low-pass filters have settled, real-time values in the "red" channel $L_R$' and the "infrared" channel $L_{IR}$' are stored. Subsequently, the system phase shift $\varphi$ is calculated on the basis of equation (13). The red LED is enabled again, and after a waiting time, until low-pass filters have settled, the normal operating mode is resumed.

The normal operating mode in this case means applying equation (21) with the previously determined phase shift $\varphi$. The correction calculation described above can be performed for example by the CPU 28.

Another method to overcome signal cross-coupling between "red" and "infrared" channels in accordance with the present invention is to use an all-pass filter anywhere in the signal path between photoreceiver and demodulator. The all-pass filter has the function of a phase shifter. This all-pass filter should have a phase shift 6 at the excitation base frequency $f_{LED}$ which yields to:

$$\delta + \varphi = n * 360° \tag{22}$$

with n = 1,2,....

Such a filter would be ideally realized in the digital domain with programmable filter constants. The phase characteristics of such a all-pass filter in the digital domain can be adjusted according to a measured or determined system phase shift. In this case, the phase measurement procedure would be as follows.

In the beginning, the normal operating mode is interrupted, the red LED is disabled, the infrared LED is continuously driven, and the all-pass function is switched off (by-pass etc.). Thereafter there is a waiting time for some milliseconds until the low-pass filters have settled. After this waiting time, the real-time values in the "red" channel $L_R$' and the "infrared" channel $L_{IR}$' are stored. On the basis of these stored values and equation (13) the system phase shift $\varphi$ is calculated.

Subsequently, the phase shift $\delta$ which the all-pass filter should have is determined according equation (22). On the basis of this determined phase shift filter, the appropriate filter constants for this type of all-pass filter are selected. After having enabled the red LED again and having waited, until low-pass filters have settled, the normal operating mode is resumed. The normal operating mode in this case means activating the all-pass function in the signal flow with the determined characteristics in order to produce a phase shift $\delta$ of the received electromagnetic waves in the signal path between photoreceiver and modulator.

Besides the method to measure the phase of the incoming signal relative to the transmitted electromagnetic waves as described above there exist other methods to measure this phase. A common step is always to change at least one of the LED's intensity by a known amount. The procedure explained above is a special case where the red LED's intensity is set to 0. This gives a best measurement resolution and makes calculation easier.

However, it is understood that any known change of one or a combination of the two LED's intensities could be used to determine the phase. An advantage of switching the intensity only to a reduced level, like 0.5, would be the possibility to continue with a normal $SpO_2$ measurement without interruption.

Referring to Fig. 13, a further method to determine the system phase shift $\varphi$ is described. In this measurement, the difference in the zero-crossing between the incoming signal A(t) and the LED drive signal $J_R$(t) and $J_{IR}$(t) is measured before demodulation, rather than measuring the amplitudes of the incoming signals on the low-pass filtered side. In Fig. 13, the incoming signal A(t) is shown as a sinusoidal wave form. The "infrared" excitation wave is shown as a quare waveform $J_{IR}$(t). As can be seen from Fig. 13, the phase difference $\varphi$ of this two waveforms which corresponds to the system phase shift $\varphi$ is determined on the basis of a negative zero-crossing of both these signals. The phase shift determined by this method can be used in the same way as the phase shift determined on the basis of equation (13).

It will be appreciated by those skilled in the art that the principle of sinusoidal demodulation as disclosed herein is also applicable to multi-wavelength oximetry. If a third and forth LED is used, a further LED modulation frequency has to be selected. In general, for n LEDs, n/2 modulation frequencies have to be used, just because each frequency channel can contain 2 independent information data in quadrature mode. The only condition for these different modulation frequencies is that they are offset by more than the physiological bandwidth of the blood pulse.

It is also clear that the sinusoidal demodulation can be applied in a two LED pulse oximeter without the quadrature concept, but with only the sinusoidal demodulation, if there is a different modulation frequency chosen for each LED.

The great advantage of interference immunity of this system may also be applied to other sensoric measurements where ambient noise is an issue, not only in medical instrumentation. It is always possible where an excitation is used for stimulation. Examples are impedance measurements in bridges like pressure strain gauges or spectrometer devices with chopped sources.

**Claims**

1. Method for measuring medical parameters of a patient by irradiation of electromagnetic waves into a sample (40) and for measurement and subsequent analysis of the electromagnetic waves which have passed through said sample (40), said method comprising the steps of:

    (1.1) generating first and second modulation signals of the same frequencies and having a first phase difference of substantially 90°;
    (1.2) irradiating a first electromagnetic wave of a first wavelength into said sample (40) under control of said first modulation signal;
    (1.3) irradiating a second electromagnetic wave of a second wavelength different from the first one into said sample (40) under control of said second modulation signal;
    (1.4) receiving electromagnetic waves (A(t)) of both wavelengths which have passed through said sample (40);
    (1.5) demodulating signals repesentative of the received electromagnetic waves (A(t)) by multiplying the same with a first sinusoidal demodulation signal and with a second sinusoidal demodulation signal having the first phase difference with respect to said first sinusoidal signal, said first and second sinusoidal demodulation signals having the same frequency as said first and second modulation signals, such as to generate a first and a second demodulated signal ($D_R(t)$, $D_{IR}(t)$); and
    (1.6) analysing said demodulated signals;
    characterized by
    the step of determining a system phase shift ($\varphi$) which is introduced by the system between the modulated electromagnetic waves which are irradiated into said sample (40) and the signals representing the received electromagnetic waves,
    wherein the first and the second sinusoidal demodulation signals have a phase difference ($\sigma$) relative to the first and second modulation signals corresponding to said system phase shift ($\varphi$).

2. Method according to claim 1, comprising the step of adjusting said phase difference ($\sigma$) of the first and second sinusoidal demodulation signals relative to the first and second modulation signals in accordance with said determined system phase shift ($\varphi$).

3. Method for measuring medical parameters of a patient by irradiation of electromagnetic waves into a sample (40) and for measure subsequent analysis of the electromagnetic waves which have passed through said sample (40), said method comprising the steps of:

    (3.1) generating first and second modulation signals of the same frequencies and having a first phase difference of substantially 90 °;
    (3.2) irradiating a first electromagnetic wave of a first wavelength into said sample (40) under control of said first modulation signal;
    (3.3) irradiating a second electromagnetic wave of a second wavelength different from the first one into said sample (40) under control of said second modulation signal;
    (3.4) receiving electromagnetic waves (A(t)) of both wavelengths which have passed through said sample (40);
    (3.5) demodulating signals repesentative of the received electromagnetic waves by multiplying the same with a first sinusoidal demodulation signal and with a second sinusoidal demodulation signal having the first phase difference with respect to said first sinusoidal signal, said first and second sinusoidal demodulation signals having the same frequency as said first and second modulation signals, such as to generate a first and a second demodulated signal ($D_R(t)$, $D_{IR}(t)$);
    characterized by the steps of:
    (3.6) determining a system phase shift ($\varphi$) which is introduced by the system between the modulated electromagnetic waves which are irradiated into said sample (40) and the signals representing the received electromagnetic waves; and
    (3.7) analysing said demodulated signals taking into account said system phase shift.

4. Method according to claim 3, wherein said analysing step comprises the step of:

    correcting said demodulated signals ($D_R(t)$, $D_{IR}(t)$) on the basis of said determined system phase shift ($\varphi$).

5. Method according to claim 3 or 4, wherein said analysing step comprises the steps of:

calculating a matrix [C] based on the system phase shift according to

$$[C] = \begin{bmatrix} \cos\varphi & -\sin\varphi \\ \sin\varphi & \cos\varphi \end{bmatrix} ;$$

calculating an inverse matrix [C]$^{-1}$ based on the matrix [C];
determining an output vector

$$[L] = \begin{vmatrix} L_R \\ L_{IR} \end{vmatrix}$$

based on the amplitudes $L_R$, $L_{IR}$ of the demodulated signals; and
calculating a corrected output vector $[A] = [C]^{-1} [L]$ .

6. Method according to one of the claims 1 to 5, wherein said step of determining said system phase shift ($\varphi$) includes the steps of:

reducing the amplitude of one of the first and second electromagnetic waves;
measuring of the amplitudes of the first and second demodulated signals ($D_R(t)$, $D_{IR}(t)$); and
determining the system phase shift ($\varphi$) on the basis of the measured amplitudes of the first and the second demodulated signal ($D_R(t)$, $D_{IR}(t)$).

7. Method according to one of the claims 1 to 5, wherein said step of determining said system phase shift ($\varphi$) includes the steps of:

reducing the amplitude of one of the first and second electromagnetic waves; and
determining the difference in a zero-crossing between the received electromagnetic waves and the other of the first and second electromagnetic waves.

8. Method for measuring medical parameters of a patient by irradiation of electromagnetic waves into a sample (40) and for measurement and subsequent analysis of the electromagnetic waves which have passed through said sample (40), said method comprising the steps of:

(8.1) generating first and second modulation signals of the same frequencies and having a first phase difference of substantially 90°;
(8.2) irradiating a first electromagnetic wave of a first wavelength into said sample (40) under control of said first modulation signal;
(8.3) irradiating a second electromagnetic wave of a second wavelength different from the first one into said sample (40) under control of said second modulation signal;
(8.4) receiving electromagnetic waves (A(t)) of both wavelengths which have passed through said sample (40);
characterized by the steps of:
(8.5) determining a system phase shift ($\varphi$) which is introduced by the system between the electromagnetic waves which are irradiated into said sample (40) and the received electromagnetic waves;
(8.6) generating delayed received electromagnetic waves by adding a phase shift ($\sigma$) to the received electromagnetic waves which yields, together with said system phase shift ($\varphi$) a total phase shift of the received electromagnetic waves relative to the first and second electromagnetic waves of substantially an integral multiple of 360°;
(8.7) demodulating signals representative of the delayed received electromagnetic waves by multiplying the same with a first sinusoidal demodulation signal and with a second sinusoidal demodulation signal having the first phase difference with respect to said first sinusoidal signal, said first and second sinusoidal demodulation signals having the same frequency as said first and second modulation signals, such as to generate a first and a second demodulated signal ($D_R(t)$, $D_{IR}(t)$); and

(8.8) analysing said demodulated signals ($D_R(t)$, $D_{IR}(t)$).

9. Method according to claim 8, wherein said phase shift ($\sigma$) is added to the received electromagnetic waves by a digital all pass filter comprising programmable filter constants.

10. Method according to claim 8 or 9, wherein said phase shift ($\sigma$) is adjusted in accordance with the steps of:

reducing the amplitude of one of the first and second electromagnetic waves;
measuring of the amplitudes of the first and second demodulated signals ($D_R(t)$, $D_{IR}(t)$);
determining the system phase shift ($\varphi$) on the basis of the measured amplitudes of the first and second demodulated signals; and
adjusting the phase shift ($\sigma$) to yield together with the system phase shift ($\varphi$) a phase shift of an integral multiple of 360°.

11. Method according to claim 6, 7 or 10, wherein the reducing of the amplitude of one of the first and second electromagnetic waves comprises turning off the amplitude of this electromagnetic wave.

12. Method according to one of claims 1 to 11, wherein the frequency of said first and second modulation signals is basically f=275 Hertz.

13. Method according to one of claims 1 to 12, wherein said first and second modulation signals are square-wave signals.

14. Method according to one of claims 1 to 11, wherein said first and second modulation signals are sinusoidal signals.

15. Method according to one of claims 1 to 14 comprising the step of low-pass filtering the demodulated signals ($D_R(t)$, $D_{IR}(t)$).

16. Method according to one of claims 1 to 15 comprising the step of band-pass filtering the received electromagnetic waves before the demodulation step.

17. Method according to one of claims 1 to 16, wherein said electromagnetic waves are waves selected from the visible and/or the adjoining spectra of light, preferably red and/or infrared light.

18. Method according to one of claims 1 to 17, wherein said medical parameter is oxygen saturation.

19. Apparatus for measuring medical parameters of a patient by irradiation of electromagnetic waves into a sample (40) and for measurement and subsequent analysis of the electromagnetic waves which have passed through said sample (40), said apparatus comprising:

means (32) for generating first and second modulation signals of the same frequencies and having a first phase difference of substantially 90°;
means (33) for irradiating a first electromagnetic wave of a first wavelength into said sample (40) under control of said first modulation signal;
means (34) for irradiating a second electromagnetic wave of a second wavelength different from the first one into said sample (40) under control of said second modulation signal;
means (35) for receiving electromagnetic waves ($A(t)$) of both wavelengths which have passed through said sample (40);
means (46) for demodulating signals repesentative of the received electromagnetic waves ($A(t)$) by multiplying the same with a first sinusoidal demodulation signal and with a second sinusoidal demodulation signal having the first phase difference with respect to said first sinusoidal signal, said first and second sinusoidal demodulation signals having the same frequency as said first and second modulation signals, such as to generate a first and a second demodulated signal ($D_R(t)$, $D_{IR}(t)$); and
means (28) for analysing said demodulated signals,
characterized by

means for determining a system phase shift ($\varphi$) which is introduced by the system between the modulated electromagnetic waves which are irradiated into said sample (40) and the signals representing the received electromagnetic waves, wherein

the first and the second sinusoidal demodulation signals have a phase difference ($\sigma$) relative to the first and second modulation signals corresponding to said system phase shift.

20. Apparatus according to claim 19, comprising means for adjusting said phase difference ($\sigma$) of the first and second sinusoidal demodulation signals relative to the first and second modulation signals in accordance with said determined system phase shift ($\varphi$).

21. Apparatus for measuring medical parameters of a patient by irradiation of electromagnetic waves into a sample (40) and for measurement and subsequent analysis of the electromagnetic waves which have passed through said sample (40), said apparatus comprising:

means (32) for generating first and second modulation signals of the same frequencies and having a first phase difference of substantially 90°;
means (33) for irradiating a first electromagnetic wave of a first wavelength into said sample (40) under control of said first modulation signal;
means (34) for irradiating a second electromagnetic wave of a second wavelength different from the first one into said sample (40) under control of said second modulation signal;
means (35) for receiving electromagnetic waves (A(t)) of both wavelengths which have passed through said sample (40);
means (46) for demodulating signals repesentative of the received electromagnetic waves by multiplying the same with a first sinusoidal demodulation signal and with a second sinusoidal demodulation signal having the first phase difference with respect to said first sinusoidal signal, said first and second sinusoidal demodulation signals having the same frequency as said first and second modulation signals, such as to generate a first and a second demodulated signal ($D_R(t)$, $D_{IR}(t)$);
characterized by
means for determining a system phase shift ($\varphi$) which is introduced by the system between the modulated electromagnetic waves which are irradiated into said sample (40) and the signals representing the received electromagnetic waves; and means (28) for analysing said demodulated signals taking into account said system phase shift.

22. Apparatus according to claim 21, wherein said means (28) for analysing comprises:

means for correcting said demodulated signals ($D_R(t)$, $D_{IR}(t)$) on the basis of said determined system phase shift ($\varphi$).

23. Apparatus according to claim 21 or 22, wherein said means (28) for analysing comprises:

means for calculating a matrix [C] based on the system phase shift according to

$$[C] = \begin{vmatrix} \cos\varphi & -\sin\varphi \\ \sin\varphi & \cos\varphi \end{vmatrix} ;$$

means for calculating an inverse matrix $[C]^{-1}$ based on the matrix [C];
means for determining an output vector

$$[L] = \begin{vmatrix} L_R \\ L_{IR} \end{vmatrix}$$

based on the amplitudes $L_R$, $L_{IR}$ of the demodulated signals; and

EP 0 761 159 B1

means for calculating a corrected output vector $[A] = [C]^{-1} \cdot [L]$ .

**24.** Apparatus according to one of the claims 19 to 23, wherein said means for determining said system phase shift ($\varphi$) comprises:

means for reducing the amplitude of one of the first and second electromagnetic waves;
means for measuring of the amplitudes of the first and second demodulated signals ($D_R(t)$, $D_{IR}(t)$); and
means for determining the system phase shift ($\varphi$) on the basis of the measured amplitudes of the first and the second demodulated signal ($D_R(t)$, $D_{IR}(t)$).

**25.** Apparatus according to one of the claims 19 to 23, wherein said means for determining said system phase shift ($\varphi$) comprises:

means for reducing the amplitude of one of the first and second electromagnetic waves; and
means for determining the difference in a zero-crossing between the received electromagnetic waves and the other of the first and second electromagnetic waves.

**26.** Apparatus for measuring medical parameters of a patient by irradiation of electromagnetic waves into a sample (40) and for measurement and subsequent analysis of the electromagnetic waves which have passed through said sample (40), said apparatus comprising:

means (32) for generating first and second modulation signals of the same frequencies and having a first phase difference of substantially 90°;
means (33) for irradiating a first electromagnetic wave of a first wavelength into said sample (40) under control of said first modulation signal;
means (34) for irradiating a second electromagnetic wave of a second wavelength different from the first one into said sample (40) under control of said second modulation signal;
means (35) for receiving electromagnetic waves ($A(t)$) of both wavelengths which have passed through said sample (40);
characterized by
means for determining a system phase shift ($\varphi$) which is introduced by the system between the electromagnetic waves which are irradiated into said sample (40) and the received electromagnetic waves;
means for generating delayed received electromagnetic waves by adding a phase shift ($\sigma$) to the received electromagnetic waves which yields, together with said system phase shift ($\varphi$) a total phase shift of the received electromagnetic waves relative to the first and second electromagnetic waves of substantially an integral multiple of 360°;
means (46) for demodulating signals representative of the delayed received electromagnetic waves by multiplying the same with a first sinusoidal demodulation signal and with a second sinusoidal demodulation signal having the first phase difference with respect to said first sinusoidal signal, said first and second sinusoidal demodulation signals having the same frequency as said first and second modulation signals, such as to generate a first and a second demodulated signal ($D_R(t)$, $D_{IR}(t)$); and
means (28) for analysing said demodulated signals ($D_R(t)$, $D_{IR}(t)$).

**27.** Apparatus according to claim 26, comprising a digital all pass filter comprising programmable filter constants for adding said phase shift ($\sigma$) to the received electromagnetic waves.

**28.** Apparatus according to claim 26 or 27, further comprising:

means for reducing the amplitude of one of the first and second electromagnetic waves;
means for measuring of the amplitudes of the first and second demodulated signals ($D_R(t)$, $D_{IR}(t)$);
means for determining the system phase shift ($\varphi$) on the basis of the measured amplitudes of the first and second demodulated signals; and
means for adjusting the phase shift ($\sigma$) to yield together with the system phase shift ($\varphi$) a phase shift of an integral multiple of 360°.

**29.** Apparatus according to claim 24, 25 or 28, wherein said means for reducing of the amplitude of one of the first and second electromagnetic waves comprises means for turning off the amplitude of this electromagnetic wave.

16

**30.** Apparatus according to one of claims 19 to 29, wherein the frequency of said first and second modulation signals is basically f=275 Hertz.

**31.** Apparatus according to one of claims 19 to 30, wherein said first and second modulation signals are square-wave signals.

**32.** Apparatus according to one of claims 19 to 30, wherein said first and second modulation signals are sinusoidal signals.

**33.** Apparatus according to one of claims 19 to 32 comprising a low pass filter through which the demodulated signals are fed.

**34.** Apparatus according to one of claims 19 to 33 comprising a bandfilter connected between said means for receiving electromagnetic waves and said demodulating means.

**35.** Apparatus according to one of claims 19 to 34, wherein said electromagnetic waves are waves selected from the visible and/or the adjoining spectra of light, preferably red and/or infrared light.

**36.** Apparatus according to one of claims 19 to 35, wherein said apparatus is a pulse oximeter, and said medical parameter is oxygen saturation.

**37.** Apparatus according to one of claims 19 to 36, wherein said irradiating means (33,34) are light-emitting diodes.

**Patentansprüche**

**1.** Verfahren zum Messen medizinischer Parameter eines Patienten durch Einstrahlung elektromagnetischer Wellen in eine Probe (40) und zur Messung und anschließenden Analyse der elektromagnetischen Wellen, welche die Probe (40) durchdrungen haben, wobei das Verfahren die folgenden Schritte umfaßt:

(1.1) Erzeugung erster und zweiter Modulationssignale der gleichen Frequenzen mit einen ersten Phasenunterschied von im wesentlichen 90°;
(1.2) Einstrahlung einer ersten elektromagnetischen Welle einer ersten Wellenlänge in die Probe (40) durch Steuerung mit dem ersten Modulationssignal;
(1.3) Einstrahlung einer zweiten elektromagnetischen Welle einer zweiten von der ersten unterschiedlichen Wellenlänge in die Probe (40) durch Steuerung mit dem zweiten Modulationssignal;
(1,4) Empfang elektromagnetischer Wellen (A(t)) beider Wellenlängen, welche die Probe (40) durchdrungen haben;
(1.5) Demodulation der Signale, die für die empfangenen elektromagnetischen Wellen (A(t)) stehen, durch Multiplikation derselben mit einem ersten sinusförmigen Demodulationssignal und einem zweiten sinusförmigen Demodulationssignal, das den ersten Phasenunterschied in bezug auf das erste sinusförmige Signal aufweist, wobei die ersten und zweiten sinusförmigen Demodulationssignale die gleiche Frequenz wie die ersten und zweiten Modulationssignale aufweisen, um ein erstes und ein zweites demoduliertes Signal ($D_R(t)$, $D_{IR}(t)$) zu erzeugen, und
(1.6) Analyse der demodulierten Signale;
gekennzeichnet durch

den Schritt der Bestimmung einer Systemphasenverschiebung ($\varphi$), die von dem System zwischen den modulierten elektromagnetischen Wellen, die in die Probe (40) eingestrahlt werden, und den Signalen, die für die empfangenen elektromagnetischen Wellen stehen, eingeführt wird;
wobei die ersten und die zweiten sinusförmigen Demodulationssignale einen Phasenunterschied ($\sigma$) bezüglich der ersten und zweiten Modulationssignale besitzen, welcher der Systemphasenverschiebung ($\varphi$) entspricht.

**2.** Verfahren gemäß Anspruch 1, das den Schritt der Anpassung des Phasenunterschieds ($\sigma$) der ersten und zweiten sinusförmigen Demodulationssignale relativ zu den ersten und zweiten Modulationssignalen in Einklang mit der bestimmten Systemphasenverschiebung ($\varphi$) umfaßt.

**3.** Verfahren zum Messen medizinischer Parameter eines Patienten durch Einstrahlung elektromagnetischer Wellen in eine Probe (40) und zur der Messung folgenden Analyse der elektromagnetischen Wellen, welche die Probe (40) durchdrungen haben, wobei das Verfahren die folgenden Schritte umfaßt:

(3.1) Erzeugung erster und zweiter Modulationssignale der gleichen Frequenz mit einem Phasenunterschied von im wesentlichen 90°;

(3.2) Einstrahlung einer ersten elektromagnetischen Welle einer ersten Wellenlänge in die Probe (40) durch Steuerung mit dem ersten Modulationssignal;

(3.3) Einstrahlung einer zweiten elektromagnetischen Welle einer zweiten von der ersten unterschiedlichen Wellenlänge in die Probe (40) durch Steuerung mit dem zweiten Modulationssignal;

(3.4) Empfang elektromagnetischer Wellen (A(t)) beider Wellenlängen, welche die Probe (40) durchdrungen haben;

(3.5) Demodulation der Signale, die für empfangenen elektromagnetischen Wellen stehen, durch Multiplikation dieser mit einem ersten sinusförmigen Demodulationssignal und mit einem zweiten sinusförmigen Demodulationssignal, das den Phasenunterschied in bezug auf das erste sinusförmige Signal aufweist, und die ersten und zweiten sinusförmigen Demodulationssignale die gleiche Frequenz wie die ersten und zweiten Modulationssignale haben, um ein erstes und zweites demoduliertes Signal ($D_R(t)$, $D_{IR}(t)$) zu erzeugen; durch die folgenden Schritte gekennzeichnet:

(3.6) Bestimmung einer Systemphasenverschiebung ($\varphi$), die von dem System zwischen den modulierten elektromagnetischen Wellen, die in die Probe (40) eingestrahlt werden, und den Signalen, die für die empfangenen Wellen stehen, eingeführt wird, und

(3.7) Analyse der demodulierten Signale unter Berücksichtigung der Systemphasenverschiebung.

4.  Verfahren gemäß Anspruch 3, wobei der Analyseschritt den folgenden Schritt umfaßt:

Korrektur der demodulierten Signale ($D_R(t)$, $D_{IR}(t)$) auf der Grundlage der bestimmten Systemphasenverschiebung ($\varphi$).

5.  Verfahren gemäß Anspruch 3 oder 4, wobei der Analyseschritt die folgenden Schritte umfaßt:

Berechnung einer Matrix [C], die auf der Systemphasenverschiebung basiert, gemäß

$$C = \begin{bmatrix} \cos\varphi & -\sin\varphi \\ \sin\varphi & \cos\varphi \end{bmatrix};$$

Berechnung einer inversen Matrix $[C]^{-1}$ basierend auf Matrix [C];
Bestimmung eines Ausgabevektors

$$[L] = \begin{bmatrix} L_R \\ L_{IR} \end{bmatrix}$$

auf Grundlage der Amplituden von $L_R$, $L_{IR}$ der demodulierten Signale und
Berechnung eines korrigierten Ausgabevektors $[A] = [C]^{-1} [L]$.

6.  Verfahren gemäß einem der Ansprüche 1 bis 5, wobei der Schritt der Bestimmung der Systemphasenverschiebung ($\varphi$) die folgenden Schritte umfaßt:

Reduktion der Amplitude einer der ersten und zweiten elektromagnetischen Wellen;
Messung der Amplituden der ersten und zweiten demodulierten Signale ($D_R(t)$, $D_{IR}(t)$) und
Bestimmung der Systemphasenverschiebung ($\varphi$) auf Basis der gemessenen Amplituden der ersten und der zweiten demodulierten Signale ($D_R(t)$, $D_{IR}(t)$).

7.  Verfahren gemäß einem der Ansprüche 1 bis 5, wobei der Schritt der Bestimmung der Systemphasenverschiebung ($\varphi$) die folgenden Schritte umfaßt:

Reduktion der Amplitude einer der ersten und zweiten elektromagnetischen Wellen und

Bestimmung des Unterschieds zwischen den empfangenen elektromagnetischen Wellen und den anderen der ersten und zweiten elektromagnetischen Wellen bei einem Nulldurchgang.

8. Verfahren zum Messen medizinischer Parameter eines Patienten durch Einstrahlung elektromagnetischer Wellen in eine Probe (40) und zur Messung und anschließender Analyse der elektromagnetischen Wellen, welche die Probe (40) durchdrungen haben, wobei das Verfahren die folgenden Schritte umfaßt:

(8.1) Erzeugung erster und zweiter Modulationssignale der gleichen Frequenz mit einem Phasenunterschied von im wesentlichen 90°;

(8.2) Einstrahlung einer ersten elektromagnetischen Welle einer ersten Wellenlänge in die Probe (40) durch Steuerung mit dem ersten Modulationssignal;

(8.3) Einstrahlung einer zweiten elektromagnetischen Welle einer zweiten von der ersten unterschiedlichen Wellenlänge in die Probe (40) durch Steuerung mit dem zweiten Modulationssignal;

(8.4) Empfang elektromagnetischer Wellen (A(t)) beider Wellenlängen, welche die Probe (40) durchdrungen haben;

durch die folgenden Schritte gekennzeichnet:

(8.5) Bestimmung einer Systemphasenverschiebung ($\varphi$), die von dem System zwischen den elektromagnetischen Wellen, die in die Probe (40) eingestrahlt werden, und den empfangenen elektromagnetischen Wellen;

(8.6) Erzeugung verzögerter empfangener elektromagnetischer Wellen durch Addition einer Phasenverschiebung ($\sigma$) zu den empfangenen elektromagnetischen Wellen, die zusammen mit der Systemphasenverschiebung ($\varphi$) eine Gesamtphasenverschiebung der empfangenen elektromagnetischen Wellen bezüglich der ersten und zweiten elektromagnetischen Wellen von im wesentlichen einem ganzzahlig Vielfachen von 360° ergibt;

(8.7) Demodulation der Signale, die für die verzögerten empfangenen elektromagnetischen Wellen stehen, durch Multiplikation derselben mit einem ersten sinusförmigen Demodulationssignal und mit einem zweiten sinusförmigen Demodulationssignal, das den Phasenunterschied in bezug auf das erste sinusförmige Signal aufweist, wobei die ersten und zweiten sinusförmigen Demodulationssignale die gleiche Frequenz wie die ersten und zweiten Modulationssignale haben, um ein erstes und zweites demoduliertes Signal ($D_R(t)$, $D_{IR}(t)$) zu erzeugen, und

(8.8) Analyse der demodulierten Signale ($D_R(t)$, $D_{IR}(t)$).

9. Verfahren gemäß Anspruch 8, wobei die Phasenverschiebung ($\sigma$) zu den empfangenen elektromagnetischen Wellen durch einen digitalen Allpaßfilter mit programmierbaren Filterkonstanten addiert wird.

10. Verfahren gemäß Anspruch 8 oder 9, wobei die Phasenverschiebung ($\sigma$) in Einklang mit den folgenden Schritten angepaßt wird:

Reduktion der Amplitude einer der ersten und zweiten elektromagnetischen Wellen;

Messung der Amplituden der ersten und zweiten demodulierten Signale ($D_R(t)$, $D_{IR}(t)$);

Bestimmung der Systemphasenverschiebung ($\varphi$) auf der Grundlage der gemessenen Amplituden der ersten und zweiten demodulierten Signale und

Anpassung der Phasenverschiebung ($\sigma$), um zusammen mit der Systemphasenverschiebung ($\varphi$) eine Phasenverschiebung eines ganzzahlig Vielfachen von 360° zu ergeben.

11. Verfahren gemäß Anspruch 6, 7 oder 10, wobei die Reduktion der Amplitude einer der ersten und zweiten elektromagnetischen Wellen das Abschalten der Amplitude dieser elektromagnetischen Welle umfaßt.

12. Verfahren gemäß einem der Ansprüche 1 bis 11, wobei die Frequenz der ersten und zweiten Modulationssignale im wesentlichen f = 275 Hertz ist.

13. Verfahren gemäß einem der Ansprüche 1 bis 12, wobei die ersten und zweiten Modulationssignale Rechteckwellensignale sind.

14. Verfahren gemäß einem der Ansprüche 1 bis 11, wobei die ersten und zweiten Modulationssignale sinusförmige Signale sind.

15. Verfahren gemäß einem der Ansprüche 1 bis 14, das den Schritt der Tiefpaßfilterung der demodulierten Signale ($D_R(t)$, $D_{IR}(t)$) umfaßt.

**16.** Verfahren gemäß einem der Ansprüche 1 bis 15, das den Schritt einer Bandpaßfilterung der empfangenen elektromagnetischen Wellen vor dem Demodulationsschritt umfaßt.

**17.** Verfahren gemäß einem der Schritte 1 bis 16, wobei die elektromagnetischen Wellen aus dem sichtbaren Spektrum und/oder den angrenzenden Spektren des Lichts, vorzugsweise rot und/oder infrarot, stammen.

**18.** Verfahren gemäß einem der Ansprüche 1 bis 17, wobei der medizinische Parameter die Sauerstoffsättigung ist.

**19.** Vorrichtung zum Messen medizinischer Parameter eines Patienten durch Einstrahlung elektromagnetischer Wellen in die Probe (40) und zur Messung und anschließenden Analyse der elektromagnetischen Wellen, welche die Probe (40) durchdrungen haben, wobei die Vorrichtung folgendes umfaßt:

Mittel (32) zur Erzeugung erster und Zweiter Modulationssignale der gleichen Frequenzen mit einem ersten Phasenunterschied von im wesentlichen 90°;
Mittel (33) zur Einstrahlung einer ersten elektromagnetischen Welle einer ersten Wellenlänge in die Probe (40) durch Steuerung mit dem ersten Modulationssignal;
Mittel (34) zur Einstrahlung einer zweiten elektromagnetischen Wellen einer zweiten von der ersten unterschiedlichen Wellenlänge in die Probe (40) durch Steuerung mit dem zweiten Modulationssignal;
Mittel (35) zum Empfang elektromagnetischer Wellen (A(t)) beider Wellenlängen, welche die Probe (40) durchdrungen haben;
Mittel (46) zur Demodulation von Signalen, die für die empfangenen elektromagnetischen Wellen (A(t)) stehen, durch Multiplikation dieser mit einem ersten sinusförmigen Demodulationssignal und mit einem zweiten sinusförmigen Demodulationssignal, das den Phasenunterschied in bezug auf das erste sinusförmige Signal aufweist, wobei die ersten und zweiten sinusförmigen Demodulationssignale die gleiche Frequenz wie die ersten und zweiten Modulationssignale haben, um ein erstes und zweites demoduliertes Signal ($D_R(t)$, $D_{IR}(t)$) zu erzeugen, und
Mittel (28) zur Analyse der demodulierten Signale,
durch folgendes gekennzeichnet:
Mittel zur Bestimmung einer Systemphasenverschiebung ($\varphi$), die durch das System zwischen den modulierten elektromagnetischen Wellen, die in die Probe (40) eingestrahlt werden, und die Signale, die für die empfangenen elektromagnetischen Wellen stehen, eingeführt wird, wobei:
die ersten und zweiten sinusförmigen Demodulationssignale einen Phasenunterschied ($\sigma$) bezüglich der ersten und zweiten Modulationssignale entsprechend der Systemphasenverschiebung besitzen.

**20.** Vorrichtung gemäß Anspruch 19, der Mittel zur Anpassung des Phasenunterschieds ($\sigma$) der ersten und zweiten sinusförmigen Demodulationssignale bezüglich der ersten und zweiten Modulationssignale in Einklang mit der bestimmten Systemphasenverschiebung ($\varphi$) umfaßt.

**21.** Vorrichtung zum Messen medizinischer Parameter eines Patienten durch Einstrahlung elektromagnetischer Wellen in eine Probe (40) und zur Messung und anschließenden Analyse der elektromagnetischen Wellen, welche die Probe (40) durchdrungen haben, wobei die Vorrichtung folgendes umfaßt:

Mittel (32) zur Erzeugung erster und zweiter Modulationssignale der gleichen Frequenzen mit einem ersten Phasenunterschied von im wesentlichen 90°;
Mittel (33) zur Einstrahlung einer ersten elektromagnetischen Welle einer ersten Wellenlänge in die Probe (40) durch Steuerung mit dem ersten Modulationssignal;
Mittel (34) zur Einstrahlung einer zweiten elektromagnetischen Wellen einer zweiten von der ersten unterschiedlichen Wellenlänge in die Probe (40) durch Steuerung mit dem zweiten Modulationssignal;
Mittel (35) zum Empfang elektromagnetischer Wellen (A(t)) beider Wellenlängen, welche die Probe (40) durchdrungen haben;
Mittel (46) zur Demodulation der Signale, die für die empfangenen elektromagnetischen Wellen stehen, durch Multiplikation dieser mit einem ersten sinusförmigen Demodulationssignal und mit einem zweiten sinusförmigen Demodulationssignal, das den Phasenunterschied in bezug auf das erste sinusförmige Signal aufweist, und die ersten und zweiten sinusförmigen Demodulationssignale die gleiche Frequenz wie die ersten und zweiten Modulationssignale haben, um ein erstes und zweites demoduliertes Signal ($D_R(t)$, $D_{IR}(t)$) zu erzeugen;
gekennzeichnet durch
Mittel zur Bestimmung einer Systemphasenverschiebung ($\varphi$), die durch das System zwischen den modulierten elektromagnetischen Wellen, die in die Probe (40) eingestrahlt werden, und die Signale, die für die empfangenen elektromagnetischen Wellen stehen, eingeführt wird, und

Mittel (28) zur Analyse der demodulierten Signale, welche die Systemphasenverschiebung berücksichtigen.

22. Vorrichtung gemäß Anspruch 21, wobei die Mittel (28) zur Analyse folgendes umfassen:

Mittel zur Korrektur der demodulierten Signale ($D_R(t)$, $D_{IR}(t)$) auf Basis der bestimmten Systemphasenverschiebung ($\varphi$).

23. Vorrichtung gemäß Anspruch 21 oder 22, wobei die Mittel (28) zur Analyse folgendes umfassen:

Mittel zur Berechnung einer Matrix [C], die auf der Systemphasenverschiebung basiert, gemäß

$$C = \begin{bmatrix} \cos\varphi & -\sin\varphi \\ \sin\varphi & \cos\varphi \end{bmatrix} ;$$

Mittel zur Berechnung einer inversen Matrix $[C]^{-1}$ basierend auf Matrix [C];
Mittel zur Bestimmung eines Ausgabevektors

$$[L] = \begin{bmatrix} L_R \\ L_{IR} \end{bmatrix}$$

auf Grundlage der Amplituden von $L_R$, $L_{IR}$ der demodulierten Signale und
Mittel zur Berechnung eines korrigierten Ausgabevektors $[A] = [C]^{-1} \cdot [L]$.

24. Vorrichtung gemäß einem der Ansprüche 19 bis 23, wobei die Mittel zur Bestimmung der Systemphasenverschiebung ($\varphi$) folgendes umfassen:

Mittel zur Reduktion der Amplitude einer der ersten und zweiten elektromagnetischen Wellen;
Mittel zur Messung der Amplituden der ersten und zweiten demodulierten Signale ($D_R(t)$, $D_{IR}(t)$) und
Mittel zur Bestimmung der Systemphasenverschiebung ($\varphi$) auf Basis der gemessenen Amplituden des ersten und zweiten demodulierten Signals ($D_R(t)$, $D_{IR}(t)$).

25. Vorrichtung gemäß einem der Ansprüche 19 bis 23, wobei die Mittel zur Bestimmung der Systemphasenverschiebung ($\varphi$) folgendes umfassen:

Mittel zur Reduktion der Amplitude einer der ersten und zweiten elektromagnetischen Wellen und
Mittel zur Bestimmung des Unterschieds zwischen den empfangenen elektromagnetischen Wellen und den anderen der ersten und zweiten elektromagnetischen Wellen bei einem Nulldurchgang.

26. Vorrichtung zur Messung medizinischer Parameter eines Patienten durch Einstrahlung elektromagnetischer Wellen in eine Probe (40) und zur Messung und anschließenden Analyse der elektromagnetischen Wellen, welche die Probe (40) durchdrungen haben, wobei die Vorrichtung folgendes umfaßt:

Mittel (32) zur Erzeugung erster und zweiter Modulationssignale der gleichen Frequenzen mit einem ersten Phasenunterschied von im wesentlichen 90°;
Mittel (33) zur Einstrahlung einer ersten elektromagnetischen Welle einer ersten Wellenlänge in die Probe (40) durch Steuerung mit dem ersten Modulationssignal;
Mittel (34) zur Einstrahlung einer zweiten elektromagnetischen Welle mit einer zweiten von der ersten unterschiedlichen Wellenlänge in die Probe (40) durch Steuerung mit dem zweiten Modulationssignal;
Mittel (35) zum Empfang elektromagnetischer Wellen (A(t)) beider Wellenlängen, welche die Probe (40) durchdrungen haben;
gekennzeichnet durch
Mittel zur Bestimmung einer Systemphasenverschiebung ($\varphi$), die durch das System zwischen den elektromagnetischen Wellen, die in die Probe (40) eingestrahlt werden, und den empfangenen elektromagnetischen Wellen eingeführt wird;

Mittel zur Erzeugung verzögerter empfangener elektromagnetischer Wellen durch Addition einer Phasenverschiebung ($\sigma$) zu den empfangenen elektromagnetischen Wellen, die zusammen mit der Systemphasenverschiebung ($\varphi$) eine Gesamtphasenverschiebung der empfangenen elektromagnetischen Wellen bezüglich der ersten und zweiten elektromagnetischen Wellen im wesentlichen ein ganzzahliges Vielfaches von 360° ergeben;

Mittel (46) zur Demodulation von Signalen, die für die empfangenen elektromagnetischen Wellen stehen, durch Multiplikation dieser mit einem ersten sinusförmigen Demodulationssignal und mit einem zweiten sinusförmigen Demodulationssignal, das den ersten Phasenunterschied bezüglich des ersten sinusförmigen Signal besitzt, und die ersten und zweiten sinusförmigen Demodulationssignale die gleiche Frequenz wie die ersten und zweiten Modulationssignale besitzen, um ein erstes und ein zweites demoduliertes Signal ($D_R(t)$, $D_{IR}(t)$) zu erzeugen, und

Mittel (28) zur Analyse der demodulierten Signale ($D_R(t)$, $D_{IR}(t)$).

27. Vorrichtung gemäß Anspruch 26, der einen digitalen Allpaßfilter mit programmierbaren Filterkonstanten zur Addition der Phasenverschiebung ($\sigma$) zu den empfangenen elektromagnetischen Wellen umfaßt.

28. Vorrichtung gemäß Anspruch 26 oder 27, der des weiteren folgendes umfaßt:

Mittel zur Reduktion der Amplitude einer der ersten und zweiten elektromagnetischen Wellen;

Mittel zur Messung der Amplituden der ersten und zweiten demodulierten Signale ($D_R(t)$, $D_{IR}(t)$);

Mittel zur Bestimmung der Systemphasenverschiebung ($\varphi$) auf der Grundlage der gemessenen Amplituden der ersten und zweiten demodulierten Signale und

Mittel zur Anpassung der Phasenverschiebung ($\sigma$), um zusammen mit der Systemphasenverschiebung ($\varphi$) eine Phasenverschiebung eines ganzzahligen Vielfachen von 360° zu ergeben.

29. Vorrichtung gemäß Anspruch 24, 25 oder 28, wobei die Mittel zur Reduktion der Amplitude einer der ersten und zweiten elektromagnetischen Wellen Mittel zur Abschaltung der Amplitude dieser elektromagnetischen Welle umfassen.

30. Vorrichtung gemäß einem der Ansprüche 19 bis 29, wobei die Frequenz der ersten und zweiten Modulationssignale im wesentlichen f = 275 Hertz ist.

31. Vorrichtung gemäß einem der Ansprüche 19 bis 30, wobei die ersten und zweiten Modulationssignale Rechteckwellensignale sind.

32. Verfahren gemäß einem der Ansprüche 19 bis 30, wobei die ersten und zweiten Modulationssignale sinusförmige Signale sind.

33. Vorrichtung gemäß einem der Ansprüche 19 bis 32, der einen Tiefpaßfilter umfaßt, durch den die demodulierten Signale ($D_R(t)$, $D_{IR}(t)$) durchgeführt werden.

34. Vorrichtung gemäß einem der Ansprüche 19 bis 33, der einen Bandpaßfilter umfaßt, der zwischen die Mittel zum Empfang elektromagnetischer Wellen und den Demodulationsmitteln angeschlossen ist.

35. Vorrichtung gemäß einem der Schritte 19 bis 34, wobei die elektromagnetischen Wellen aus dem sichtbaren Spektrum und/oder den angrenzenden Spektren des Lichts, vorzugsweise rot und/oder infrarot, gewählte Wellen durch sind.

36. Vorrichtung gemäß einem der Ansprüche 19 bis 35, wobei die Vorrichtung ein Puls-Oximeter und der medizinische Parameter die Sauerstoffsättigung ist.

37. Vorrichtung gemäß einen der Ansprüche 19 bis 36, wobei die Einstrahlungsmittel (33, 34) Leuchtdioden sind.

## Revendications

1. Procédé pour mesurer des paramètres médicaux d'un patient par irradiation d'un échantillon (40) à l'aide d'ondes électromagnétiques et pour une mesure suivie d'une analyse des ondes électromagnétiques qui sont passées à travers ledit échantillon (40), ledit procédé comprenant les étapes consistant à :

(1.1) engendrer un premier et un deuxième signal de modulation de même fréquence et présentant une différence de phase sensiblement égale à 90° ;

(1.2) irradier ledit échantillon (40) à l'aide d'une première onde électromagnétique ayant une première longueur d'onde sous la commande dudit premier signal de modulation ;

(1.3) irradier ledit échantillon (40) à l'aide d'une deuxième onde électromagnétique ayant une deuxième longueur d'onde différente de la première sous la commande dudit deuxième signal de modulation ;

(1.4) recevoir des ondes électromagnétiques (A(t)) des deux longueurs d'ondes qui sont passées à travers ledit échantillon (40) ;

(1.5) démoduler les signaux représentatifs des ondes électromagnétiques reçues (A(t)) en multipliant ceux-ci par un premier signal sinusoïdal de démodulation et par un deuxième signal sinusoïdal de démodulation présentant la première différence de phase par rapport au dit premier signal sinusoïdal, lesdits premier et deuxième signaux sinusoïdaux de démodulation étant de même fréquence que lesdits premier et deuxième signaux de modulation, de manière à engendrer un premier et un deuxième signal démodulé ($D_R(t)$, $D_{IR}(t)$) ; et

(1.6) analyser lesdits signaux démodulés ;

caractérisé par

l'étape consistant à déterminer un décalage de phase ($\varphi$) du système qui est introduit par le système entre les ondes électromagnétiques modulées qui ont irradié ledit échantillon (40) et les signaux représentant les ondes électromagnétiques reçues,

dans lequel les premier et deuxième signaux sinusoïdaux de démodulation présentent une différence de phase ($\sigma$) relative au premier et deuxième signaux de modulation qui correspond au dit décalage de phase du système ($\varphi$).

2. Procédé conforme à la revendication 1, comprenant l'étape consistant à régler ladite différence de phase ($\sigma$) des premier et deuxième signaux sinusoïdaux de démodulation relativement aux premier et deuxième signaux de modulation conformément au décalage de phase ($\varphi$) déterminé pour le système.

3. Procédé pour mesurer des paramètres médicaux d'un patient par irradiation d'un échantillon (40) à l'aide d'ondes électromagnétiques et pour une mesure suivie d'une analyse des ondes électromagnétiques qui sont passées à travers ledit échantillon (40), ledit procédé comprenant les étapes consistant à :

(3.1) engendrer un premier et un deuxième signal de modulation de même fréquence et présentant une différence de phase sensiblement égale à 90° ;

(3.2) irradier ledit échantillon (40) à l'aide d'une première onde électromagnétique ayant une première longueur d'onde sous la commande dudit premier signal de modulation ;

(3.3) irradier ledit échantillon (40) à l'aide d'une deuxième onde électromagnétique ayant une deuxième longueur d'onde différente de la première sous la commande dudit deuxième signal de modulation ;

(3.4) recevoir des ondes électromagnétiques (A(t)) des deux longueurs d'ondes qui sont passées à travers ledit échantillon (40) ;

(3.5) démoduler les signaux représentatifs des ondes électromagnétiques reçues en multipliant ceux-ci par un premier signal sinusoïdal de démodulation et par un deuxième signal sinusoïdal de démodulation présentant la première différence de phase par rapport au dit premier signal sinusoïdal, lesdits premier et deuxième signaux sinusoïdaux de démodulation étant de même fréquence que lesdits premier et deuxième signaux de modulation, de manière à engendrer un premier et un deuxième signal démodulé ($D_R(t)$, $D_{IR}(t)$) ;

caractérisé par les étapes consistant à :

(3.6) déterminer un décalage de phase ($\varphi$) du système qui est introduit par le système entre les ondes électromagnétiques modulées qui ont irradié ledit échantillon (40) et les signaux représentant les ondes électromagnétiques reçues ; et

(3.7) analyser lesdits signaux démodulés en prenant en compte ledit décalage de phase du système.

4. Procédé conforme à la revendication 3, dans lequel ladite étape pour analyser comprend l'étape consistant à :

corriger lesdits signaux démodulés ($D_R(t)$, $D_{IR}(t)$) sur la base dudit décalage de phase ($\varphi$) déterminé pour le système.

5. Procédé conforme à la revendication 3 ou à la revendication 4, dans lequel ladite étape pour analyser comprend les étapes consistant à :

calculer une matrice [C] sur la base du décalage de phase du système conformément à

$$[C] = \begin{bmatrix} \cos\varphi & -\sin\varphi \\ \sin\varphi & \cos\varphi \end{bmatrix}$$

calculer une matrice inverse $[C]^{-1}$ sur la base de la matrice [C] ;
déterminer un vecteur de sortie

$$[L] = \begin{bmatrix} L_R \\ L_{IR} \end{bmatrix}$$

sur la base des amplitudes $L_R$, $L_{IR}$ des signaux démodulés ; et
calculer un vecteur de sortie corrigé $[A]=[C]^{-1}[L]$.

6.  Procédé conforme à l'une quelconque des revendications 1 à 5 dans lequel ladite étape pour déterminer ledit décalage de phase ($\varphi$) du système inclut les étapes consistant à :

    réduire l'amplitude d'une parmi les première et deuxième ondes électromagnétiques ;
    mesurer les amplitudes des premier et deuxième signaux démodulés ($D_R$(t), $D_{IR}$(t)) ; et
    déterminer le décalage de phase ($\varphi$) du système sur la base des amplitudes mesurées du premier et du deuxième signal démodulé ($D_R$(t), $D_{IR}$(t)).

7.  Procédé conforme à l'une quelconque des revendications 1 à 5, dans lequel ladite étape pour déterminer ledit décalage de phase ($\varphi$) du système inclut les étapes consistant à :

    réduire l'amplitude d'une parmi les première et deuxième ondes électromagnétiques ; et
    déterminer la différence dans un passage par zéro entre les ondes électromagnétiques reçues et l'autre parmi les première et deuxième ondes électromagnétiques.

8.  Procédé pour mesurer des paramètres médicaux d'un patient par irradiation d'un échantillon (40) à l'aide d'ondes électromagnétiques et pour une mesure suivie d'une analyse des ondes électromagnétiques qui sont passées à travers ledit échantillon (40), ledit procédé comprenant les étapes consistant à :

    (8.1) engendrer un premier et un deuxième signal de modulation de même fréquence et présentant une différence de phase sensiblement égale à 90° ;
    (8.2) irradier ledit échantillon (40) à l'aide d'une première onde électromagnétique ayant une première longueur d'onde sous la commande dudit premier signal de modulation ;
    (8.3) irradier ledit échantillon (40) à l'aide d'une deuxième onde électromagnétique ayant une deuxième longueur d'onde différente de la première sous la commande dudit deuxième signal de modulation ;
    (8.4) recevoir des ondes électromagnétiques (A(t)) des deux longueurs d'ondes qui sont passées à travers ledit échantillon (40) ;
    caractérisé par les étapes consistant à :
    (8.5) déterminer un décalage de phase ($\varphi$) du système qui est introduit par le système entre les ondes électromagnétiques qui ont irradié ledit échantillon (40) et les ondes électromagnétiques reçues ;
    (8.6) engendrer des ondes électromagnétiques reçues retardées en ajoutant un décalage de phase ($\sigma$) aux ondes électromagnétiques reçues ce qui fournit, avec ledit décalage de phase ($\varphi$) du système un décalage de phase total sensiblement égal à un multiple entier de 360° pour les ondes électromagnétiques reçues relativement aux première et deuxième ondes électromagnétiques ;
    (8.7) démoduler des signaux représentatifs des ondes électromagnétiques reçues retardées en multipliant celles-ci par un premier signal sinusoïdal de démodulation et par un deuxième signal sinusoïdal de démodulation présentant la première différence de phase par rapport au dit premier signal sinusoïdal, lesdits premier et deuxième signaux sinusoïdaux de démodulation étant de même fréquence que lesdits premier et deuxième signaux de modulation, de manière à engendrer un premier et un deuxième signal démodulé ($D_R$(t), $D_{IR}$(t)) ; et
    (8.8) analyser lesdits signaux démodulés ($D_R$(t) , $D_{IR}$(t)).

**9.** Procédé conforme à la revendication 8, dans lequel ledit décalage de phase ($\sigma$) est ajouté aux ondes électromagnétiques reçues par un filtre passe tout numérique comprenant des constantes de filtre programmables.

**10.** Procédé conforme à la revendication 8 ou à la revendication 9 dans lequel le décalage de phase ($\sigma$) est réglé conformément aux étapes consistant à :

réduire l'amplitude d'une parmi les première et deuxième ondes électromagnétiques ;
mesurer les amplitudes des premier et deuxième signaux démodulés (DR(t), DIR(t)) ;
déterminer le décalage de phase ($\varphi$) du système sur la base des amplitudes mesurées pour les premier et deuxième signaux démodulés ; et
régler le décalage de phase ($\sigma$) pour fournir avec le décalage de phase ($\varphi$) du système un décalage de phase égal à un multiple entier de 360°.

**11.** Procédé conforme à l'une des revendications 6, 7 ou 10, dans lequel la réduction de l'amplitude de l'une parmi les première et deuxième ondes électromagnétiques consiste à éteindre l'amplitude de cette onde électromagnétique.

**12.** Procédé conforme à l'une quelconque des revendications 1 à 11, dans lequel la fréquence desdits premier et deuxième signaux de modulation est en gros f = 275 Hz.

**13.** Procédé conforme à l'une quelconque des revendications 1 à 12, dans lequel les premier et deuxième signaux de modulation sont des signaux carrés.

**14.** Procédé conforme à l'une quelconque des revendications 1 à 11, dans lequel les premier et deuxième signaux de modulation sont des signaux sinusoïdaux.

**15.** Procédé conforme à l'une quelconque des revendications 1 à 14 comprenant l'étape consistant à filtrer les signaux démodulés ($D_R(t)$, $D_{IR}(t)$ par un passe bas.

**16.** Procédé conforme à l'une quelconque des revendications 1 à 15 comprenant l'étape consistant à filtrer les ondes électromagnétiques reçues par un passe bande avant l'étape de démodulation.

**17.** Procédé conforme à l'une quelconque des revendications 1 à 16, dans lequel lesdites ondes électromagnétiques sont des ondes sélectionnées dans le spectre de la lumière visible et/ ou dans le spectre contigu, de préférence une lumière rouge et/ ou infrarouge.

**18.** Procédé conforme à l'une quelconque des revendications 1 à 17, dans lequel ledit paramètre médical est une saturation en oxygène.

**19.** Dispositif pour mesurer des paramètres médicaux d'un patient par irradiation d'un échantillon (40) à l'aide d'ondes électromagnétiques et pour une mesure suivie d'une analyse des ondes électromagnétiques qui sont passées à travers ledit échantillon (40), ledit dispositif comprenant :

un moyen (32) pour engendrer un premier et un deuxième signal de modulation de même fréquence et présentant une première différence de phase sensiblement égale à 90° ;
un moyen (33) pour irradier ledit échantillon (40) à l'aide d'une première onde électromagnétique ayant une première longueur d'onde sous la commande dudit premier signal de modulation ;
un moyen (34) pour irradier ledit échantillon (40) à l'aide d'une deuxième onde électromagnétique ayant une deuxième longueur d'onde différente de la première sous la commande dudit deuxième signal de modulation ; un moyen (35) pour recevoir des ondes électromagnétiques (A(t)) des deux longueurs d'ondes qui sont passées à travers ledit échantillon (40) ;
un moyen (46) pour démoduler des signaux représentatifs des ondes électromagnétiques reçues (A(t)) en multipliant ceux-ci par un premier signal sinusoïdal de démodulation et par un deuxième signal sinusoïdal de démodulation présentant la première différence de phase par rapport au dit premier signal sinusoïdal, lesdits premier et deuxième signaux sinusoïdaux de démodulation étant de même fréquence que lesdits premier et deuxième signaux de modulation, de manière à engendrer un premier et un deuxième signal démodulé ($D_R(t)$, $D_{IR}(t)$) ; et un moyen (28) pour analyser lesdits signaux démodulés ;
caractérisé par
un moyen pour déterminer un décalage de phase ($\varphi$) du système qui est introduit par le système entre les ondes électromagnétiques modulées qui ont irradié ledit échantillon (40) et les signaux représentant les ondes électromagnétiques reçues,

les premier et deuxième signaux sinusoïdaux de démodulation présentant une différence de phase ($\sigma$) relative aux premier et deuxième signaux de modulation qui correspond au dit décalage de phase du système ($\varphi$).

20. Dispositif conforme à la revendication 19, comprenant un moyen pour régler ladite différence de phase ($\varphi$) des premier et deuxième signaux sinusoïdaux de démodulation relativement aux premier et deuxième signaux de modulation conformément au décalage de phase ($\varphi$) déterminé pour le système.

21. Dispositif pour mesurer des paramètres médicaux d'un patient par irradiation d'un échantillon (40) à l'aide d'ondes électromagnétiques et pour une mesure suivie d'une analyse des ondes électromagnétiques qui sont passées à travers ledit échantillon (40), ledit dispositif comprenant

un moyen (32) pour engendrer un premier et un deuxième signal de modulation de même fréquence et présentant une première différence de phase sensiblement égale à 90° ;

un moyen (33) pour irradier ledit échantillon (40) à l'aide d'une première onde électromagnétique ayant une première longueur d'onde sous la commande dudit premier signal de modulation ;

un moyen (34) pour irradier ledit échantillon (40) à l'aide d'une deuxième onde électromagnétique ayant une deuxième longueur d'onde différente de la première sous la commande dudit deuxième signal de modulation ;

un moyen (35) pour recevoir des ondes électromagnétiques (A(t)) des deux longueurs d'ondes qui sont passées à travers ledit échantillon (40) ;

un moyen (46) pour démoduler des signaux représentatifs des ondes électromagnétiques reçues en multipliant ceux-ci par un premier signal sinusoïdal de démodulation et par un deuxième signal sinusoïdal de démodulation présentant la première différence de phase par rapport au dit premier signal sinusoïdal, lesdits premier et deuxième signaux sinusoïdaux de démodulation étant de même fréquence que lesdits premier et deuxième signaux de modulation, de manière à engendrer un premier et un deuxième signal démodulé ($D_R(t)$, $D_{IR}(t)$) ;

caractérisé par :

un moyen pour déterminer un décalage de phase ($\varphi$) du système qui est introduit par le système entre les ondes électromagnétiques modulées qui ont irradié ledit échantillon (40) et les signaux représentant les ondes électromagnétiques reçues ; et

un moyen (28) pour analyser lesdits signaux démodulés en prenant en compte ledit décalage de phase du système.

22. Dispositif conforme à la revendication 21, dans lequel ledit moyen (28) pour analyser comprend :

un moyen pour corriger lesdits signaux démodulés ($D_R(t)$, $D_{IR}(t)$) sur la base dudit décalage de phase ($\varphi$) déterminé pour le système.

23. Dispositif conforme à la revendication 21 ou à la revendication 22, dans lequel ledit moyen (28) pour analyser comprend :

un moyen pour calculer une matrice [C] sur la base du décalage de phase du système conformément à

$$[C] = \begin{bmatrix} \cos\varphi & -\sin\varphi \\ \sin\varphi & \cos\varphi \end{bmatrix}$$

un moyen pour calculer une matrice inverse $[C]^{-1}$ sur la base de la matrice [C] ;
un moyen pour déterminer un vecteur de sortie

$$[L] = \begin{bmatrix} L_R \\ L_{IR} \end{bmatrix}$$

sur la base des amplitudes $L_R$, $L_{IR}$ des signaux démodulés ; et
un moyen pour calculer un vecteur de sortie corrigé $[A]=[C]^{-1}[L]$.

**24.** Dispositif conforme à l'une quelconque des revendications 19 à 23 dans lequel ledit moyen pour déterminer ledit décalage de phase ($\varphi$) du système comprend :

un moyen pour réduire l'amplitude d'une parmi les première et deuxième ondes électromagnétiques ;
un moyen pour mesurer les amplitudes des premier et deuxième signaux démodulés ($D_R(t)$, $D_{IR}(t)$) ; et
un moyen pour déterminer le décalage de phase ($\varphi$) du système sur la base des amplitudes mesurées du premier et du deuxième signal démodulé ($D_R(t)$, $D_{IR}(t)$).

**25.** Dispositif conforme à l'une quelconque des revendications 19 à 23, dans lequel ledit moyen pour déterminer ledit décalage de phase ($\varphi$) du système comprend :

un moyen pour réduire l'amplitude d'une parmi les première et deuxième ondes électromagnétiques ; et
un moyen pour déterminer la différence de passage par zéro entre les ondes électromagnétiques reçues et l'autre parmi les première et deuxième ondes électromagnétiques.

**26.** Dispositif pour mesurer des paramètres médicaux d'un patient par irradiation d'un échantillon (40) à l'aide d'ondes électromagnétiques et pour une mesure suivie d'une analyse des ondes électromagnétiques qui sont passées à travers ledit échantillon (40), ledit dispositif comprenant :

un moyen (32) pour engendrer un premier et un deuxième signal de modulation de même fréquence et présentant une première différence de phase sensiblement égale à 90° ;
un moyen (33) pour irradier ledit échantillon (40) à l'aide d'une première onde électromagnétique ayant une première longueur d'onde sous la commande dudit premier signal de modulation ;
un moyen (34) pour irradier ledit échantillon (40) à l'aide d'une deuxième onde électromagnétique ayant une deuxième longueur d'onde différente de la première sous la commande dudit deuxième signal de modulation ;
un moyen (35) pour recevoir des ondes électromagnétiques ($A(t)$) des deux longueurs d'ondes qui sont passées à travers ledit échantillon (40) ;
caractérisé par :
un moyen pour déterminer un décalage de phase ($\varphi$) du système qui est introduit par le système entre les ondes électromagnétiques qui ont irradié ledit échantillon (40) et les ondes électromagnétiques reçues ;
un moyen pour engendrer des ondes électromagnétiques reçues retardées en ajoutant un décalage de phase ($\sigma$) aux ondes électromagnétiques reçues ce qui fournit, avec ledit décalage de phase ($\varphi$) du système un décalage de phase total sensiblement égal à un multiple entier de 360° pour les ondes électromagnétiques reçues relativement aux première et deuxième ondes électromagnétiques ;
un moyen (46) pour démoduler des signaux représentatifs des ondes électromagnétiques reçues retardées en multipliant celles-ci par un premier signal sinusoïdal de démodulation et par un deuxième signal sinusoïdal de démodulation présentant la première différence de phase par rapport au dit premier signal sinusoïdal, lesdits premier et deuxième signaux sinusoïdaux de démodulation étant de même fréquence que lesdits premier et deuxième signaux de modulation, de manière à engendrer un premier et un deuxième signal démodulé ($D_R(t)$, $D_{IR}(t)$) ; et
un moyen pour analyser lesdits signaux démodulés ($D_R(t)$, $D_{IR}(t)$).

**27.** Dispositif conforme à la revendication 26, comprenant un filtre passe tout numérique comportant des constantes de filtre programmables pour ajouter ledit décalage de phase ($\sigma$) aux ondes électromagnétiques reçues.

**28.** Dispositif conforme à la revendication 26 ou à la revendication 27, comprenant en outre :

un moyen pour réduire l'amplitude d'une parmi les première et deuxième ondes électromagnétiques ;
un moyen pour mesurer les amplitudes des premier et deuxième signaux démodulés ($D_R(t)$, $D_{IR}(t)$) ;
un moyen pour déterminer le décalage de phase ($\varphi$) du système sur la base des amplitudes mesurées pour les premier et deuxième signaux démodulés ; et
un moyen pour régler le décalage de phase ($\sigma$) pour obtenir avec le décalage de phase ($\varphi$) du système un décalage de phase égal à un multiple entier de 360°.

**29.** Dispositif conforme à l'une des revendications 24, 25 ou 28, dans lequel ledit moyen pour réduire l'amplitude de l'une parmi les première et deuxième ondes électromagnétiques comporte un moyen pour éteindre l'amplitude de cette onde électromagnétique.

**30.** Dispositif conforme à l'une quelconque des revendications 19 à 29, dans lequel la fréquence desdits premier et deuxième signaux de modulation est en gros f = 275 Hz.

**31.** Dispositif conforme à l'une quelconque des revendications 19 à 30, dans lequel les premier et deuxième signaux de modulation sont des signaux carrés.

**32.** Dispositif conforme à l'une quelconque des revendications 19 à 30, dans lequel les premier et deuxième signaux de modulation sont des signaux sinusoïdaux.

**33.** Dispositif conforme à l'une quelconque des revendications 19 à 32 comprenant un filtre passe bas à travers lequel les signaux démodulés ($D_R(t)$, $D_{IR}(t)$) sont fournis.

**34.** Dispositif conforme à l'une quelconque des revendications 19 à 33 comprenant un filtre de bande connecté entre ledit moyen de réception des ondes électromagnétiques et ledit moyen de démodulation.

**35.** Dispositif conforme à l'une quelconque des revendications 19 à 34, dans lequel lesdites ondes électromagnétiques sont des ondes sélectionnées dans le spectre de la lumière visible et/ ou dans le spectre contigu, de préférence une lumière rouge et/ ou infrarouge.

**36.** Dispositif conforme à l'une quelconque des revendications 19 à 35, dans lequel ledit dispositif est un oxymètre à impulsions, et ledit paramètre médical est une saturation en oxygène.

**37.** Dispositif conforme à l'une quelconque des revendications 19 à 36, dans lequel le moyen pour irradier (33, 34) est constitué de diodes émettrices de lumière.

FIG.1
PRIOR ART

EP 0 761 159 B1

FIG.2

FIG.3a

FIG.3b

FIG.3c

PRIOR ART

30

FIG. 4

FIG. 5

EP 0 761 159 B1

**FIG.6**

EP 0 761 159 B1

FIG. 7

FIG. 8

FIG.9

EP 0 761 159 B1

FIG. 10

FIG. 11

FIG. 12

FIG. 13